# EUROPÄISCHE PATENTSCHRIFT

(11) **EP 2 666 181 B1**
(45) Veröffentlichungstag und Bekanntmachung des Hinweises auf die Patenterteilung: **18.03.2015**
(21) Anmeldenummer: 12700606.2
(22) Anmeldetag: 17.01.2012
(51) Int. Cl.: H01J 37/32, B05D 3/14, C23C 16/04

(54) **PLASMA-BEHANDLUNGSVORRICHTUNG ZUR HERSTELLUNG VON BESCHICHTUNGEN**
PLASMA TREATMENT DEVICE FOR PRODUCING COATINGS
DISPOSITIF DE TRAITEMENT PLASMA POUR LA FABRICATION DE REVÊTEMENTS

(30) Priorität: 20.01.2011 DE 102011009057
(43) Veröffentlichungstag der Anmeldung: 27.11.2013
(73) Patentinhaber: Schott AG, 55122 Mainz (DE)
(72) Erfinder: BICKER, Matthias, 55126 Mainz (DE); LOHMEYER, Manfred, 55299 Nackenheim (DE); BAUCH, Hartmut, 55278 Mommenheim (DE)
(74) Vertreter: Blumbach Zinngrebe
(86) Internationale Anmeldenummer: PCT/EP2012/000162
(87) Internationale Veröffentlichungsnummer: WO 2012/097972

(56) Entgegenhaltungen:
- EP-A1- 2 147 988
- WO-A1-2011/029628
- US-A1- 2010 298 738

## Beschreibung

Die Erfindung betrifft allgemein das technische Gebiet der Behandlung von Oberflächen und Beschichtungen mit Plasmen. Insbesondere betrifft die Erfindung eine Vorrichtung und ein Verfahren zur Behandlung von Schichten mit einer gegenüber dem äußeren Atmosphärendruck abgeschlossenen Plasma-Zone.

Plasmabehandlungsverfahren zur Behandlung von hohlkörperförmigen Substraten sind aus der DE 10 2005 040 266 A1, der DE 103 14 064 A1, der DE 102 24 395 A1 und der US 2010/298738 A1 bekannt. Gemäß der DE 103 14 067 A1 und der DE 102 24 395 A1 wird eine Reaktionskammer dadurch gebildet, dass ein hülsenförmiges Wandelement mit einem Substratträger oder Kammerboden dichtend zusammengeführt wird. Dabei ist das zu behandelnde Substrat auf dem Kammerboden, beziehungsweise dem Substratträger angeordnet. Die DE 10 2005 040 266 A1 beschreibt eine Anordnung, bei welcher das Prozessgas den Behandlungsbereich zwischen gegenüberliegenden Enden der zu beschichtenden Pharmaverpackung durchströmt. Auch die US2010/298738 A1 offenbart eine Vorrichtung und ein Verfahren zur Behandlung von Behältern mittels einer im Innern des Behälters angeordneten Elektrodenanordnung. Die in diesen Druckschriften beschriebenen Verfahren dienen insbesondere dazu, eine Plasmabeschichtung innenseitig auf die Substrate aufzubringen.

Aus dem Stand der Technik sind Silikonöl-basierte Gleitschichten bekannt, die in vielfältigen Industriezweigen ihren Einsatz gefunden haben. Speziell für parenterale Pharma-Verpackungen wie Spritzen und Karpulen werden in der Regel Silikonöle als Gleitschichtsysteme eingesetzt. So beschreibt die US 4767414 A ein Verfahren zum Reduzieren der statischen und dynamischen Reibung zwischen Gleitflächen durch Aufbringen eines Gleitfilms auf zumindest eine der Oberflächen. Dabei wird ein niedermolekulares Silikonöl auf eine der Oberflächen aufgetragen. Das Silikonöl und die Oberfläche werden plasmabehandelt.

Manche biopharmazeutische Produkte sind allerdings Silikonöl-intolerant, so dass diese keine hinreichende Stabilität in konventionellen silikonisierten Packmitteln, wie vorbefüllten silikonisierten Spritzen, besitzen. Eine bekannte Ursache für diese Silikonöl-Intoleranz liegt darin, dass Silikonöl zur Bildung von Partikeln neigt, wodurch eine Silikonöl-Partikel-induzierte Protein-Aggregation ausgelöst wird.

Marktseitig werden daher aktuell neue Packmittellösungen gesucht, um Biopharmazeutika lagerstabil in einem silikonfreien, vorbefüllten Spritzensystem ("PFS = prefillable syringe") aufbewahren zu können. Benötigt wird dazu ein neues Gleitschichtsystem, das sowohl die Anforderungen für die tribologischen Eigenschaften für die Reibpartner Spritzenkörper / Stopfen erfüllt und zugleich nur eine geringe Oberflächen-Wechselwirkung mit den Biomolekülen der Wirkstoffformulierung aufweist.

Die US 2004/0231926 A1 beschreibt ein Verfahren zur Herstellung einer Gleitschicht, bei dem die Gleitschicht bei Atmosphärendruck unter anderem unter Verwendung eines Atmosphärendruck-Plasmas ausgehärtet wird. Neben Silikonölbasierten Schichten können auch Perfluorpolyether-basierte Gleitschichten hergestellt werden. Die Losbrechkraft, beziehungsweise die Haftreibung der letzteren Schichten erweist sich allerdings als höher verglichen mit ausgehärteten Silikonöl-Schichten. Zudem kann es bei einer Atmosphärendruck-Behandlung, insbesondere bei Atmosphärendruck-Plasma-Behandlung, zum erhöhten Eintrag von Gasen, insbesondere auch von Reaktionsprodukten des Plasmas in die Schicht kommen.

Aus der DE 10 2009 041 132 A1 ist ein Verfahren zur Herstellung eines pharmazeutischen Packmittels bekannt, bei welchem in einem ersten Prozessschritt ein silikonfreies Fluid auf der Innenseite eines Pharmapackmittels, wie insbesondere einem Spritzenkörper oder einer Karpule aufgetragen und der aufgetragene Film in einem zweiten Schritt mittels einer Niederdruck-Glimmentladung vernetzt wird.

Der Erfindung liegt die Aufgabe zugrunde, eine rationelle Herstellung von Gleitschichten mit verbesserten Eigenschaften, insbesondere im Hinblick auf die Gleiteigenschaften, der Lagerungsstabilität und der Verträglichkeit mit dem Inhalt der Packmittel zu ermöglichen.

Erfindungsgemäß ist dazu eine Vorrichtung zur Plasma-Behandlung von Behältern vorgesehen, welche - zumindest einer Behälteraufnahme-Vorrichtung mit einer Behälteraufnahme-Kammer umfasst, die wenigstens ein offenes Ende aufweist, durch welches ein zu behandelnder Behälter einführbar ist, so dass eine Öffnung des Behälters zum offenen Ende der Behälteraufnahme-Kammer weist; Weiterhin ist ein Schließelement vorgesehen, welches mit der Behälteraufnahme-Vorrichtung über eine Hubvorrichtung zusammenführbar ist, so dass beim Verschließen mittels der Hubvorrichtung durch aufeinander zu Bewegen von Schließelement und Behälteraufnahme-Vorrichtung ein aufgenommener Behälter an einer Öffnung des Behälters gegenüber dem äußeren Atmosphärendruck abgedichtet wird. Zur Plasmabehandlung ist eine Einrichtung zum Erzeugen wenigstens einer gegenüber dem äußeren Atmosphärendruck abgeschlossenen Plasma-Zone mit zwei Elektroden vorgesehen, über die ein elektrisches oder elektromagnetisches Feld im Inneren eines aufgenommenen und abgedichteten Behälters erzeugbar ist, wobei die Einrichtung zum Erzeugen einer Plasma-Zone weiterhin eine Einrichtung zum Entfernen des atmosphärischen Restgases aus dem Behälter über eine gasdichte Verbindung am Schließelement umfasst;
Die Vorrichtung weist außerdem zumindest ein Behandlungswerkzeug auf, das für wenigstens einen Teilschritt der Oberflächen-Behandlung der Behälter mit dem offenen Ende der Behälteraufnahme-Kammer der Behälteraufnahme-Vorrichtung zusammenführbar ist, wobei das Behandlungswerkzeug eine Vorrichtung zum Aufbringen eines organischen Films auf der Innenseite eines Behälters umfasst.
Vorzugsweise ist die Vorrichtung als integrierte Mehr-Kammer Vorrichtung zum Herstellen von Silikon-freien Gleitschichten ausgebildet.

Die Vorrichtung umfasst demgemäß einen Plasma-Reaktor mit einem Substratträger in Gestalt der Behälteraufnahmevorrichtung und ein Schließelement, das mit dem Substratträger über eine Hubvorrichtung verbunden wird. Weiterhin umfasst die Plasma-Behandlungsvorrichtung wenigstens ein Behandlungswerkzeug, beispielsweise in Form einer Sprühdüse oder Elektrode, das aktiv zum Substratträger über eine Hubvorrichtung verfahren wird.

In einer bevorzugten Ausführung der Erfindung umfasst die Vorrichtung dabei wenigstens eine Station zum Aufsprühen eines vorzugsweise silikonfreien organischen Fluids mit entsprechend zum Aufsprühen des Fluids ausgebildeten Behandlungswerkzeugen und eine damit verkettete PlasmaBehandlungsstation.

Im Speziellen umfasst eine erfindungsgemäße Vorrichtung zur Plasma-Behandlung von Werkstücken, insbesondere von Behältern, insbesondere der innenseitigen Plasma-Behandlung der Behälter zumindest eine Behälteraufnahme-Vorrichtung mit einer Behälteraufnahme-Kammer, die ein offenes Ende aufweist, durch welches ein zu behandelnder Behälter einführbar ist, so dass eine-Öffnung des Behälters zum offenen Ende der Behälteraufnahme-Kammer weist.

Es ist wenigstens ein Schließelement vorgesehen, wobei das Schließelement mit der Behälteraufnahme-Vorrichtung über eine Hubvorrichtung zusammenführbar ist, so dass beim Verschließen mittels der Hubvorrichtung durch aufeinander zu Bewegen von Schließelement und Behälteraufnahme-Vorrichtung ein aufgenommener Behälter an einer Öffnung abgedichtet wird.

Die Plasmabehandlung kann gemäß einer ersten Ausführungsform der Erfindung in einem Atmosphärendruck-Plasma erfolgen. In diesem Fall bedeutet das Entfernen des atmosphärischen Restgases das Austauschen des atmosphärischen Restgases mit einem Prozessgas.

Besonders bevorzugt wird die Plasmabehandlung aber in einem Niederdruck-Plasma durchgeführt. Die größeren freien Weglängen der Plasmateilchen ermöglichen höhere Teilchenenergien und führen damit zu einer besonders wirksamen Plasmabehandlung, die insbesondere in einer Vernetzung des organischen Films bestehen kann.

Gemäß einer bevorzugten Weiterbildung der Erfindung ist dazu die Vorrichtung zur Erzeugung einer Nierderdruck-Plasma-Zone mit zwei Elektroden ausgebildet, über die ein elektrisches oder elektromagnetisches Feld im Inneren eines aufgenommenen und evakuierten Behälters erzeugbar ist, wobei die Einrichtung zum Erzeugen eines Niederdruck-Plasmas weiterhin eine Einrichtung zum Evakuieren des Behälters über eine Vakuumverbindung am Schließelement umfasst.

In einer weiteren Ausführungsform der Erfindung ist die Vorrichtung dazu eingerichtet, Behälter mit einem proximalen und einem distalen offenen Ende, beziehungsweise einem ersten und einem zweiten, gegenüberliegenden Ende auf der Innenseite der Behälter mit einem Plasma zu behandeln. Gedacht ist hierbei insbesondere an Spritzen- und Karpulenkörper. Vorzugsweise umfasst die Vorrichtung dazu wenigstens zwei bewegliche Schließelemente, um die beiden Öffnungen zu verschließen. Beispielsweise wird durch Zusammenführen von einem ersten Schließelement am proximalen oder ersten Ende und einem zweiten Schließelement am distalen oder zweiten Ende mit der Behälteraufnahme-Kammer in gasdichter Verbindung mit den Behältern ein gegenüber dem äußeren Atmosphärendruck abgeschlossener Plasmareaktor gebildet.

Die Hubvorrichtung kann beispielsweise mit einer Linearachse oder einem pneumatischem Hubzylinder realisiert werden.

Weiterhin ist für die Plasmabehandlung eine Einrichtung zum Erzeugen einer vom äußeren Atmosphörendruck abgeschlossenen Plasma-Zone, in einer bevorzugten Form als eine Niederdruck-Plasma-Zone mit zwei Elektroden, über die ein elektrisches oder elektromagnetisches Feld im Inneren eines aufgenommenen und evakuierten Behälters erzeugbar ist, vorgesehen.

Unter einer gegenüber dem äußeren Atmosphärendruck abgeschlossenen Plasmazone wird im Sinne der Erfindung ein Plasma in einem räumlich abgetrennten Bereich verstanden, welches in einem Prozessgas erzeugt wird, bei dem der oder die Partialdrücke des Prozessgases in Relation zum äußeren Atmosphärendruck eine Differenz von wenigstens 1 mbar, vorzugsweise von wenigstens 5 mbar, besonders bevorzugt von wenigstens 100 mbar aufweisen.

Unter einem Niederdruck-Plasma wird im Sinne der Erfindung ein Plasma verstanden, welches in einem Prozessgas erzeugt wird, bei dem der insgesamt im Behälter zur Plasmabehandlung eingestellte Prozessdruck vorzugsweise im Bereich von 0,01 mbar bis 50 mbar, besonders bevorzugt im Bereich von 0,5 mbar bis 20 mbar, insbesondere bevorzugt in einem Bereich von 1 mbar bis 10 mbar liegt.

Außerdem ist zumindest ein Behandlungswerkzeug vorgesehen, das für wenigstens einen Teilschritt der Oberflächen-Behandlung mit dem offenen Ende der Behälteraufnahme-Kammer der Behälteraufnahme-Vorrichtung zusammenführbar ist. Zusammenführen heißt dabei vorzugsweise, dass das Behandlungswerkzeug entweder mit der Behälteraufnahme-Vorrichtung verbunden wird oder in die Behälteraufnahme-Vorrichtung eingeführt wird, um die Oberflächen-Behandlung durchzuführen.

Das Behandlungswerkzeug kann insbesondere eine Vorrichtung zum Aufbringen eines organischen Films auf der Innenseite eines mit dem Plasma zu behandelnden Behälters umfassen. Vorzugsweise wird der organische Film mittels einer Sprühdüse als organischer Flüssigkeitsfilm aufgesprüht.

Mit der Abdichtung eines in die Behälteraufnahme-Vorrichtung eingeführten Behälters an der mittels der Hubvorrichtung mit dem Schließelement in Verbindung gebrachten Behälteröffnung wird eine Abgrenzung der Plasmazone gegenüber dem äußeren Atmosphärendruck erzielt. Durch das Verschließen des Behälters mit dem Schließelement wird auf diese Weise eine Plasma-Behandlungskammer gebildet, dessen Wandung die Innenwandung des zu behandelnden Behälters darstellt.

Das mit der erfindungsgemäßen Vorrichtung durchführbare Verfahren zur innenseitigen Plasmabehandlung von Behältern basiert dementsprechend auf folgenden Prozeßschritten:
- ein Behälter mit Öffnung wird in das offene Ende einer Behälteraufnahme-Kammer einer Behälteraufnahme-Vorrichtung eingesetzt, so dass die Öffnung des Behälters zum offenen Ende der Behälteraufnahme-Kammer weist,
- ein Behandlungswerkzeug wird mit dem offenen Ende der Behälteraufnahme-Kammer zusammengeführt,
- ein organischer Films wird mittels des Behandlungswerkzeugs auf der Innenseite des in die Behälteraufnahme-Kammer eingesetzten Behälters aufgebracht,
- der Behälter wird an dessen Öffnung mittels eines Schließelements geschlossen, welches durch die Betätigung einer Hubvorrichtung mit der Behälteraufnahme-Vorrichtung zusammengeführt wird, und der Behälter wird mit dem Schließelement gegenüber dem außeren Atmosphärendruck abgedichtet,
- das atmosphärische Restgas des Behälterinneren wird mittels einer dafür geeigneten Einrichtung über eine gasdichte Verbindung am Schließelement entfernt,
- es wird eine Plasmazone im Inneren des Behälters erzeugt und der organische Film mit dem Plasma behandelt.

Besonders bevorzugt wird wie gesagt eine Niederdruck-Plasmabehandlung durchgeführt. Gemäß dieser Weiterbildung der Erfindung erfolgen also folgende Prozessschritte:
- Aufbringen eines organischen Films mittels des Behandlungswerkzeugs auf der Innenseite des in die Behälteraufnahme-Kammer eingesetzten Behälters,
- Schließen des Behälters an dessen Öffnung mittels eines Schließelements, welches durch die Betätigung einer Hubvorrichtung mit der Behälteraufnahme-Vorrichtung zusammengeführt wird, und Abdichten des Behälters mit dem Schließelement,
- Evakuieren des Behälterinneren mittels einer Einrichtung zum Evakuieren des Behälters über eine Vakuumverbindung am Schließelement,
- Erzeugen eines Niederdruck-Plasmas im Inneren des evakuierten Behälters und Behandeln des organischen Films mit dem Niederdruck-Plasma.

Es hat sich gezeigt, dass für die Herstellung von organischen, vernetzten Gleitfilmen folgende Nachteile bei einer Plasmabehandlung in Atmosphärendruck-Plasmen auftreten:

Aufgrund des geringen Energieeintrags von Atmosphärendruckplasmen ist nur eine schwache Vernetzung der Gleitschicht erreichbar und die Oberflächenfunktionalisierung ist aufgrund der geringen Anregungsenergien ineffektiv, da diese aufgrund der geringen Anregungsenergie der Teilchen des Atmosphärendruck-Plasmas nur sehr schwach ausgeprägt wird. Ferner besitzen die durch das Atmosphärendruck-Plasma angeregten Teilchen nur eine geringe Energie, so dass diese nur in die äußersten Oberflächenlagen der Gleitschichten eindringen und wirken können. Ein weiterer Nachteil des Atmosphärendruckplasma-Verfahrens ist, dass die Prozesskosten aufgrund der benötigten hohen Gasflüsse steigen. Die benötigten Gasflüsse liegen um einen Faktor 10-1000 höher als bei Niederdruck-Glimmentladungsprozessen und es muss ein teures Inertgas wie Helium verwendet werden, wodurch die Verbrauchskosten steigen. Bei Atmosphärendruckplasmen kann es zudem aufgrund des Kontakts der Plasmazone zur Umgebungsluft zur Bildung von unerwünschten toxischen Gasen, wie Stickoxiden oder Ozon kommen. Ein Atmosphärendruck-Plasma besitzt gewöhnlich eine inhomogenere Plasmazone. In den meisten Atmosphärendruck-Plasmen gibt es lokale Zonen mit sehr hoher Plasma-Intensität, wie beispielsweise bei einer Corona-Entladung unter Atmosphärendruck als auch bei einer atmosphärischen dielektrisch behinderten Entladung, bei der es in vielen Fällen zur Ausbildung von Filamenten kommt. Daher können leicht lokale Inhomogenitäten der Schicht durch die Oberflächenbehandlung entstehen.

Demgegenüber ergeben sich folgende Vorteile der erfindungsgemäßen Niederdruck-Plasma-Behandlung:

Es ist eine homogenere Oberflächenbehandlung durch eine homogene Entladungszone innerhalb des Behälters im Vergleich zu Atmosphärendruck-Plasmen erzielbar, insbesondere können Streamer und Filamente in der Entladung leicht vermieden werden. Es ergibt sich eine effizientere und bessere Vernetzung der Gleitschicht aufgrund des höheren Energieeintrags der Teilchen im Nieder-Glimmentladungsprozess im Vergleich zum Atmosphärendruckplasma. Die Eindringtiefe der Teilchen des Plasmas in die Gleitschicht ist höher, wodurch auch tiefere Lagen der Gleitschicht vernetzt werden.

Zersetzungsprodukte und volatile Verbindungen werden durch das Evakuieren entfernt. Weiterhin ist auch eine simultane Vor-Sterilisation möglich und aufgrund des höheren Energieeintrags effizienter als bei Atmosphärendruckplasmen.

Besonders vorteilhaft ist es, eine Lanze als Bestandteil der Einrichtung zum Erzeugen eines Niederdruck-Plasmas vorzusehen, welche in das Behälterinnere eines in der Behälteraufnahme-Kammer aufgenommenen Behälters, insbesondere durch das Zusammenführen mit dem Schließelement einführbar ist. Die Lanze kann zumindest eines der folgenden Merkmale aufweisen:
- die Lanze bildet eine der beiden Elektroden, wobei die Elektrode als Innenelektrode ausgebildet ist;
- die Lanze bildet einen Gasverteiler zur Einführung und Verteilung von Prozessgas für das Niederdruck-Plasma;
- die Lanze bildet eine Gasabsaug-Vorrichtung zum Absaugen von Prozessgas.

Durch die Kombination von Lanze und beweglichem Schließelement kann auf überraschend einfache Weise erreicht werden, dass gleichzeitig das Behandlungswerkzeug in Form der Lanze in den Behälter eingeführt und positioniert wird und zugleich eine Abdichtung der Plasma-Kammer als auch ein elektrische Abgrenzung der Plasmazone erfolgt.

Auf weitere überraschende Weise kann simultan zur Abdichtung der Plasmakammer mit einem beweglichem Schließelement der Anschluss an die Medienversorgung erfolgen:

In dieser bevorzugten Ausführungsform enthält das eine bewegliche Schließelement wenigstens einen offenen Kanal mit einem Anschluss an eine Medienversorgung über eine gasdichte Leitung. Die Medienversogung kann in Form von Prozessgas oder Vakuum erfolgen, so dass die gasdichte Leitung an einen Druckluftbehälter mit Prozessgas oder eine Vakuumpumpe angeschlossen ist. Vorzugsweise umfasst die Vorrichtung ferner ein gasdichtes, schaltbares Ventil in der Gasleitung, um die Medienversorgung mit der Prozesskammer zu verbinden oder abzutrennen.

In einer besonders bevorzugten Ausführungsform ist der offene Kanal des einen beweglichen Schließelements mit einer Vakuumpumpe verbunden.

Eine weitere Ausführungsform sieht dabei ferner ein zweites bewegliches Schließelement vor, das an einen Druckluftbehälter für Prozessgas angeschlossen ist.

Als organischer Film wird besonders bevorzugt ein vernetzbarer Film aufgebracht, der dann im Niederdruck-Plasma vernetzt werden kann. Demgemäß ist dazu vorgesehen, dass die Vorrichtung zum Aufbringen eines organischen Films einen Vorratsbehälter mit einer in einem Plasma vernetzbaren organischen Substanz umfasst.

Prozessgase für die Plasmabehandlung sind vorzugsweise Argon, Helium, Xenon, Sauerstoff, Stickstoff oder Gemische dieser Gase, besonders bevorzugt Argon, Sauerstoff, Stickstoff oder ein Gemisch aus diesen Gasen.

Als Materialien oder Bestandteile des organischen Films, gegebenenfalls auch weiterer Schichten, insbesondere zur Verbesserung der Gleitwirkung oder zusätzlich auch der Haftungsverbesserung des organischen Films auf dem Behälter können folgende Materialien verwendet werden:
- Perfluorpolyether (PFPE) wie die unter den Handelsnamen Fomblin M03, M30, M100, Y, Z vertriebenen Produkte, Fluorolink-Verbindungen,
- Perfluorsiloxane,
- Gleitschichten, die PTFE-Partikel enthalten,
- Öle, wie Mineralöl, Pflanzenöl, Tier-basiertes Öl,
- synthetische fluide Kohlenwasserstoffe,
- fluide fluorierte oder chlorierte Kohlenwasserstoffe,
- organische Ester, wie Fettsäureester,
- Polyphenylether,
- Phosphorsäureester,
- Polyethylen- oder Polyalkylenglykole,
- Polyalphaolefine,
- Polyaromaten, wie Alkylbenzene,
- Polyuretane,
- Squalene.

Besonders bevorzugt werden silikon-freie organische Filme, welche Fluoralkyl- und/oder Ethylen-Gruppen aufweisen, vorzugsweise in Form eines Fluids mit fluorierten oder perfluorierten Polyethern, im gegenüber dem äußeren Atmosphärendruck abgeschlossenen Plasma vernetzt.

Bevorzugt werden dabei Perfluorpolyetherverbindungen, besonders bevorzugt mit folgender Molekülstruktur für den vernetzbaren organischen Film verwendet:
i) R1-(0-CF-R-CF₂)ₚ - (0-CF₂)_{q}-R2 mit p/q im Bereich von 0,1 bis 1,0 und mit R = -CF₃ oder R= -F, mit
ii) funktionellen Gruppen R1, R2 ausgewählt aus folgender Menge:

   -CF₃, -F, -OH, -CₓH_{y}-OH, -CH₂-OH, CH₂(OCH₂CH₂)ᵣOH,

   -CH₂OCH₂CH (OH) CH₂OH, -CH₂OCH₂-Piperonyl.

Gemäß dieser Weiterbildung der Erfindung wird demgemäß allgemein ein Perfluorpolyether-haltiger organischer Film aufgetragen und im Niederdruck-Plasma vernetzt.

Weiterhin ist es günstig, unmittelbar vor dem Aufbringen des organischen Films die Behälter zu waschen und zu trocknen, insbesondere bei der Beschichtung von Glasbehältern, beispielsweise von Glasspritzenkörpern oder Glas-Karpulen. Vorzugsweise liegt dabei der Zeitversatz zwischen dem Reinigungsschritt und dem nachfolgenden Aufbringen des organischen Films unterhalb von 10 Minuten, vorzugsweise unterhalb von 5 Minuten, besonders bevorzugt unterhalb von 30 Sekunden.

Gemäß einer bevorzugten Ausführungsform der Erfindung werden die Behandlungsschritte räumlich getrennt. Dazu sind zumindest zwei Behandlungsstationen vorgesehen. Besonders praktisch ist es dabei, die Behälteraufnahme-Vorrichtung als Transportbehälter zu verwenden. Dazu ist eine Transporteinrichtung zum Transport der Behälteraufnahme-Vorrichtung zwischen den Behandlungsstationen vorgesehen. Eine erste der Behandlungsstationen umfasst die Vorrichtung zum Aufbringen des organischen Films auf der Innenseite eines Behälters. Eine zweite der Behandlungsstationen ist in Transportrichtung der Transporteinrichtung der ersten Behandlungsstation nachgeordnet und umfasst die Einrichtung zum Erzeugen einer gegenüber dem äußeren Atmosphärendruck abgeschlossenen Plasma-Zone, vorzugsweise zum Erzeugen eines Niederdruck-Plasmas. Somit wird der zuvor in der ersten Behandlungsstation aufgebrachte organische Film in der zweiten Behandlungsstation mit dem Niederdruckplasma behandelt, wobei diese Behandlung, wie oben beschrieben insbesondere eine Vernetzung des Films beinhalten kann.

Es ist besonders günstig für einen einfachen Bewegungsablauf in der erfindungsgemäßen Vorrichtung, wenn auch das Behandlungswerkzeug ebenso wie das Schließelement mit einer Hubvorrichtung verfahrbar ist, um das Behandlungswerkzeug mit der Behälteraufnahme-Vorrichtung zusammenzuführen. Insbesondere kann ein Behandlungswerkzeug, wie etwa eine Sprühdüse damit in den Behälter eingeführt werden.

Um eine gleichmäßige Beschichtung auf der Innenseite der Behälter zu bewirken, hat es sich weiterhin als günstig erwiesen, wenn die Behälter zumindest während des Schrittes des Aufbringens des organischen Films mit ihrer Öffnung, beziehungsweise bei mehreren Öffnungen mit einer ihrer Öffnungen nach unten weisend gehaltert werden. Es ist dabei besonders bevorzugt, die Behälter mit ihrer Längsache senkrecht orientiert zu haltern, jedoch ist auch eine schräge Halterung möglich. Die Längsachse sollte dabei aber vorzugsweise zwischen 9 Uhr und 15 Uhr, also nicht weiter als 45° von der Senkrechten orientiert sein. Bei der Beschichtung mit der organischen Schicht ergeben sich dadurch eine bessere Uniformität des Films auf dem Behälter. So wird bei Spritzenkörpern als Behältern verhindert, dass der aufgesprühte Film sich im Schulterbereich des Spritzenkörpers anreichert bzw. in den Luer-Kanal / die Nadel-Mündung läuft und diese verstopft.

Weist der Behälter zwei gegenüberliegende Öffnungen auf und hat an den Öffnungen unterschiedliche Innendurchmesser, wie es insbesondere auch bei Spritzenkörpern und Karpulenkörpern der Fall ist, ist es dabei besonders von Vorteil wenn die Behälter mit der Öffnung mit dem größeren Innendurchmesser nach unten weisend gehalten werden. Demgemäß ist in Weiterbildung der Erfindung vorgesehen, dass die Vorrichtung zur Behandlung von vorzugsweise Zylinder-symmetrischen Behältern mit einer Längsachse, einem distalen Ende E1 und einem proximalen Ende E2 mit unterschiedlichen Innendurchmessern ausgebildet ist, wobei der Innendurchmesser D_{E1} des distalen Endes kleiner ist, als der Innendurchmesser D_{E2} des proximalen Endes, und wobei durch die Vorrichtung die Behälter während des Sprühens mit dem Ende E1 mit dem kleineren Innendurchmesser nach oben zeigend entlang ihrer Längsachse im Bereich von 0° bis 45° zur Senkrechten, vorzugsweise im Bereich von -5° bis 5° orientiert werden.

Auch bei der Plasmabehandlung wird der Behälter vorzugsweise mit einer seiner Öffnungen, dabei bei Behältern mit mehreren Öffnungen mit unterschiedlichen Öffnungsquerschnitten also vorzugsweise mit der Öffnung mit dem größeren Innendurchmesser nach unten weisend, mit einer Orientierung der Längsachse nicht weiter als 45° von der Senkrechten gehaltert. Dadurch kann verhindert werden, dass Partikel in den Hohlkörper hinein fallen können. Zudem wird bei einer Beibehaltung der Orientierung zwischen den Schritten des Beschichtens und Plasmabehandelns die Handhabung der Behälter, beziehungsweise damit einhergehend die Konstruktion der Vorrichtung vereinfacht.

Die Erfindung ist insbesondere geeignet, um Pharmapackmittel mit Innenbeschichtungen zu versehen. Insbesondere kann die Erfindung dazu verwendet werden, silikonfreie, vernetzte organische Gleitschichten herzustellen, welche das Gleiten eines Kolbens, etwa in einem Pharmabehälter in Form einer Spritze oder Karpule verbessert und eine gute Verträglichkeit mit der Wirkstoff-Füllung der Spritze aufweisen. Im Falle einer Spritze oder Karpule erfolgt die Abdichtung durch das Schließelement an der Kolbenöffnung. Insbesondere bei Spritzen erweist sich die Orientierung der Spritzen mit der Flansch-Öffnung nach unten als sehr vorteilhaft, um das Eindringen von Partikeln zu verhindern.

Die Erfindung wird nachfolgend genauer und unter Bezugnahme auf die beigeschlossenen Figuren näher erläutert. Dabei verweisen in den Figuren gleiche Bezugszeichen auf gleiche oder entsprechende Elemente.

Es zeigen:
- Fig. 1: schematisch eine erste Ausführungsform einer Vorrichtung zur Plasma-Behandlung von Spritzenkörpern,
- Fig. 2: die Vorrichtung mit geschlossenem Reaktor,
- Fig. 3: Details der Vorrichtung mit Behälteraufnahme-Vorrichtung und Schließelement.
- Fig. 4: eine weitere Ausführungsform der Erfindung,
- Fig. 5: ein Ausführungsbeispiel einer Vorrichtung 1 zur Plasmabehandlung mit Zentriervorrichtungen,
- Fig. 6: Teile der in Fig. 4 gezeigten Ausführungsform mit geschlossenem Plasmareaktor,
- Fig. 7: einen Ausschnitt einer Behälteraufnahme-Vorrichtung,
- Fig. 8: eine weitere Ausführungsform einer Behälteraufnahme-Vorrichtung mit zwei elektrisch separierten Außenelektroden mit Öffnungen zur Aufnahme mehrerer Spritzenkörper und mit beweglichem Schließelement,
- Fig. 9, Fig. 10: weitere Ausführungsformen einer Vorrichtung zur Plasma-Behandlung von Spritzenkörpern,
- Fig. 11: Messergebnisse der Losbrechkraft und der mittleren Gleitkraft für die Bewegung eines Kolbens in mit einer Gleitschicht beschichteten Spritzenkörpern.

Die nachfolgend anhand der Figuren beschriebenen Vorrichtungen und entsprechend mit diesen Vorrichtungen durchführbaren Plasma-Behandlungen von Behältern betreffen insbesondere die Herstellung von Silikon-freien Gleitschichten für Pharma-Spritzen und Karpulen. Der Spritzenkörper der Spritze oder Karpule kann sowohl aus Glas, wie auch aus Kunststoff gefertigt sein. Auf beiden Werkstoffen ist eine stabile Beschichtung mit einer silikonfreien Gleitschicht möglich. Die nachfolgende Beschreibung bezieht sich teilweise speziell auf die Behandlung von Spritzenkörper. Die Erfindung ist aber gleichermaßen auf Karpulen anwendbar, die ebenfalls wie Spritzen einen Behälter in Form eines Zylinders zur Aufnahme eines Kolbens aufweisen.

Als Kunststoffe eignen sich für die Oberflächenbeschichtung oder -behandlung besonders Zyklo-Olefin-Copolymere (COC) und Zykloolefinpolymere (COP), aber alternativ auch Polyethylene in Form von HDPE, MDPE und LDPE als auch Polypropylene. Bevorzugte Gläser sind Borosilikatgläser.

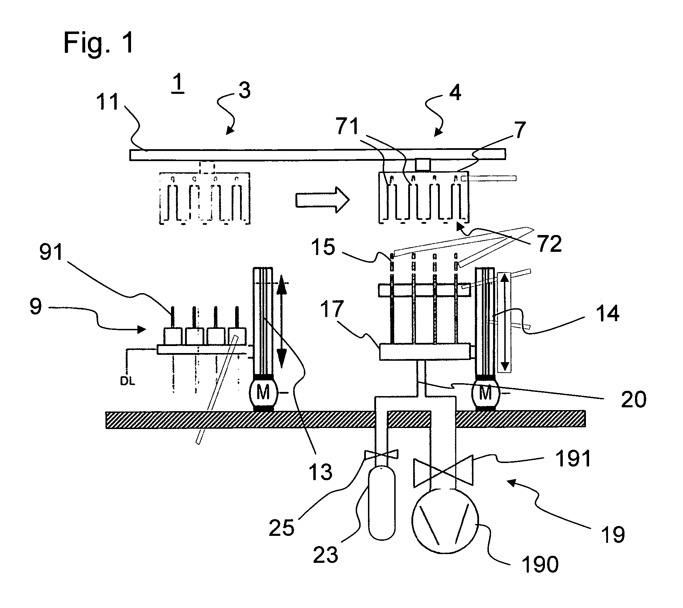

Die Vorrichtung 1, wie sie in Fig. 1 gezeigt ist, umfasst zwei Behandlungsstationen 3, 4 für die Prozess-Schritte des Aufsprühens und des Aushärtens der organischen Filme im Plasma vorgesehen. An der Behandlungsstation 3 wird der organische Film auf der Innenseite der Behälter, hier speziell auf der Innenwandung des Zylinders von Spritzenkörpern aufgetragen.

In der Behandlungsstation 4 erfolgt dann die Vernetzung der aufgetragenen Filme in einem Niederdruck-Plasma. Vorzugsweise wird, ohne Beschränkung auf das dargestellte Ausführungsbeispiel, für die Plasmabehandlung eine Niederdruck-Glimmentladung eingesetzt.

Mit der erfindungsgemäßen Vorrichtung wird eine Beschichtung mit organischen Filmen, wie insbesondere organischen Gleitschichten in einer schnellen Abfolge in einem integrierten Verfahren ermöglicht, bei welchem in einer ersten Behandlungsstation 3 ein Vorbeschichten (vorzugsweise durch Aufsprühen) mit einem organischen Fluid und einer anschließenden Vernetzung mittels eines Plasma-Behandlungsprozesses in der zweiten Behandlungsstation 4 ermöglicht wird, wobei die Dauer der Verfahrensschritte des Fluid-Beschichtens in der Behandlungsstation 3 und der Plasma-Vernetzung in Behandlungsstation 4 insgesamt weniger als 30 Sekunden, vorzugsweise weniger als 10s, besonders bevorzugt weniger als 5s beträgt.

Die Vorrichtung 1 umfasst eine Behälteraufnahme-Vorrichtung 7 mit mehreren Behälteraufnahme-Kammern 71, die ein offenes Ende 72 aufweisen, durch welche die zu behandelnden Spritzenkörper eingesetzt werden, so dass deren Kolbenöffnung jeweils zum offenen Ende 72 der Behälteraufnahme-Kammer weist. Ohne Beschränkung auf das gezeigte Beispiel ist es in Weiterbildung der Erfindung besonders günstig, wenn die Behälteraufnahmevorrichtung wenigstens zwei vorzugsweise wenigstens vier Behandlungskammern zur simultanen Behandlung der Behälter umfasst. In einer solchen Behälteraufhnahmevorrichtung können dann dementsprechend wenigstens zwei, vorzugsweise vier Behälter simultan mittels separierter Plasma-Behandlungszonen behandelt werden.

Die Behälteraufnahme-Vorrichtung 7 wird mittels einer Transporteinrichtung 11 horizontal von der Behandlungsstation 3 zur neben dieser Behandlungsstation 3 angeordneten Behandlungsstation 4 bewegt.

Die Vorrichtung 1 mit der Transporteinrichtung kann dabei allgemein, ohne Beschränkung auf das in Fig. 1 gezeigte Beispiel als getaktete lineare Batch-Anlage oder getakteter Rundläufer oder Runddrehtisch ausgestaltet sein.

Die Behandlungsstation 3 dient dem Auftrag des organischen Films auf der Innenseite des Spritzen- oder Karpulenzylinders, wobei die in Transportrichtung der Transporteinrichtung 11 der Behandlungsstation 3 nachgeordnete Behandlungsstation 4 eine Einrichtung zum Erzeugen eines Niederdruck-Plasmas in den Spritzenkörpern umfasst, um den aufgetragenen organischen Film zu verfestigen, insbesondere zu vernetzen und an die SubstratOberfläche anzubinden.

Das Behandlungswerkzeug 9 der Behandlungsstation 3 umfasst eine Vielzahl von Sprühdüsen 91. Diese werden mittels einer Hubvorrichtung 13 in die Spritzenkörper eingeführt, welche in der Behälteraufnahme-Vorrichtung 7 aufgenommen und in mittels der Transporteinrichtung 11 über den Sprühdüsen positioniert sind.

Um den Flüssigkeitsfilm des organischen Fluids auf der Innenoberfläche des Spritzen- oder Karpulenzylinders zu verteilen, können in Weiterbildung der Erfindung allgemein die Sprühdüsen 91 während des Aufsprühens mittels der Hubvorrichtung 13 in axialer Richtung entlang der Spritzenoder Karpulenzylinder bewegt werden. Die Bewegung der Sprühdüsen erfolgt vorzugsweise mit einer Geschwindigkeit im Bereich von 1mm/s bis 200mm/s.

Generell ist es, ohne Beschränkung auf die spezielle Ausbildung des in Fig. 1 gezeigten Ausführungsbeispiels, günstig, den organischen Film unter axialer Bewegung eines Sprühkopfes, beziehungsweise einer Sprühdüse 91 mit einer Hubgeschwindigkeit im Bereich von 1 mm/s bis 1000mm/s, vorzugsweise im Bereich von 5mm/s bis 200mm/s, besonders bevorzugt im Bereich von 8mm/s bis 50mm/s durchzuführen. Das Aufsprühen erfolgt vorzugsweise mit einer Rate höher als 0,1 µl /s, besonders bevorzugt höher als 0,3 µl/s.

Bevorzugte Fluid-Mengen für das Aufbringen des organischen Films liegen im Bereich von 0,004 µl / cm² bis 2,8 µl / cm², besonders bevorzugt im Bereich von 0,009 µl / cm² bis 0,22 µl / cm². Damit können geschlossene Fluidfilme erzielt werden, die andererseits aber auch hinreichend dünn sind, um in der Niederdruck-Plasmabehandlung eine durchgehende Vernetzung zu erzielen.

Weiterhin ist es günstig, die Gleitschicht aus einem Fluid mit einer Viskosität im Bereich von 1 bis 10.000 Centi-Stokes mit einem Viskositätsindex (gemäß dem Standard ASTM D 2270) größer als 80, vorzugsweise größer als 100, besonders bevorzugt größer als 150 herzustellen. Dies ermöglicht einerseits die Erzeugung kleiner Flüssigkeitströpfchen beim Aufsprühen und andererseits wird ein Verlaufen des Films und damit das Entstehen von Schichtdicken-Ungleichheiten unterdrückt.

Ist der Behandlungsschritt des Aufbringens eines organischen Films mittels des Behandlungswerkzeugs 9 auf der Innenseite der in die Behälteraufnahme-Kammern 71 eingesetzten Spritzen oder Karpulen abgeschlossen, wird das Behandlungswerkzeug 9 mittels der Hubvorrichtung 13 von der Behälteraufnahme-Vorrichtung 7 weggefahren, so dass die Behälteraufnahme-Vorrichtung 7 nun mittels der Transporteinrichtung 11 zur Behandlungsstation 4 befördert werden kann.

Die Behandlungsstation 4 umfasst für jede Behälteraufnahmekammer 71 jeweils eine Lanze 15. Die Lanzen 15 sind an einem Schließelement 17 angeordnet.

Allgemein, ohne Beschränkung auf das gezeigte Ausführungsbeispiel ist das Behandlungswerkzeug, wie etwa die Lanze 15 oder die Innenelektrode fest mit dem Schließelement verbunden. Wird, wie auch bei dem gezeigten Ausführungsbeispiel eine Innenelektrode verwendet, um das Feld für das Niederdruck-Plasma zu erzeugen, ist es im Allgemeinen günstig, die Innenelektrode mindestens bis zur Hälfte der Behälterlänge in den Behälter einzuführen. Dies ist von Vorteil, um ein hinreichend starkes Feld im Wesentlichen im ganzen Volumen des Behälters zu erzielen.

Das Schließelement 17 ist wiederum an einer Hubvorrichtung 14 angeordnet und mit der Hubvorrichtung 14 ebenso wie die Sprühdüsen 9 der Behandlungsstation 3 axial entlang der Spritzen- oder Karpulenzylinder verfahrbar.

Die Behandlungsstation 4 umfasst eine Einrichtung zum Erzeugen eines Niederdruck-Plasmas mit zwei Elektroden, über die ein elektrisches oder elektromagnetisches Feld im Inneren eines aufgenommenen und evakuierten Spritzenzylinders erzeugbar ist. Zur Plasmabehandlung des aufgetragenen organischen Films wird zunächst das Schließelement 17 mit der Behälteraufnahme-Vorrichtung 7 mittels der Hubvorrichtung 14 zusammengeführt, wobei bei diesem Verschließen mittels der Hubvorrichtung 14 durch aufeinander zu Bewegen von Schließelement 17 und Behälteraufnahme-Vorrichtung 7 die Spritzen oder Karpulen an deren Öffnung mit dem Schließelement abgedichtet werden. Weiterhin werden die Lanzen 15 beim Zusammenführen von Behälteraufahme-Vorrichtung 7 und Schließelement 17 in die Spritzenzylinder eingeführt.

Am Schließelement 17 ist eine Einrichtung 19 zum Evakuieren der Spritzenzylinder über eine Vakuumverbindung 20 am Schließelement 17 angeschlossen. Die Einrichtung 19 zum Evakuieren umfasst eine Vakuumpumpe 190 und ein Ventil 191. Die Vakuumverbindung 20 kann ebenso wie die Prozessgas-Zuführung durch flexible Verbindungen, vorzugsweise gasdichte Schläuche realisiert werden.

Das gasdichte Leitungssystem, bei dem in Fig. 1 gezeigten Beispiel also auch die Vakuumverbindung, kann allgemein mit flexiblen Schläuchen realisiert werden. Auch die Zuführung des Prozessgases kann mit einem flexiblen Schlauch realisiert werden. Die flexiblen Schläuche erlauben die Bewegung des Schließelements 17.

Das Ventil 191 kann auch eine Drosselklappe sein oder eine Drosselklappe umfassen. Ohne Beschränkung auf das in Fig. 1 gezeigte Ausführungsbeispiel ist in Weiterbildung der Erfindung vorgesehen, eine Druckregelung in der Plasmazone im Inneren der zu behandelnden Behälter mittels einer solchen, an der Ableitung zur Vakuumpumpe vorhandenen Drosselklappe und mittels einer auf die Drosselklappe wirkenden Druckregeleinrichtung zu regeln.

Die Ausführungsform gemäß Fig. 1 basiert, ohne Beschränkung auf die spezielle Ausgestaltung des Ausführungsbeispiels unter anderem auf eine Bewegung entlang zweier Bewegungsachsen, einer horizontalen Bewegung der Behälteraufnahme-Vorrichtung und einer vertikalen Bewegung, mit welcher die Behandlungswerkzeuge eingeführt und die Spritzenkörper auf der Seite der Kolbenöffnungen mit dem Schließelement zusammengeführt und abgedichtet werden.

Ein Prozessgas-Behälter 23 mit Dosierventil 24 dient dazu, die evakuierten Spritzenzylinder unter niedrigem Druck mit geeignetem Prozessgas zu füllen. Das Prozessgas wird über die Lanzen 15 in die Spritzenzylinder eingeführt. Das Evakuieren kann ebenfalls, über separate Öffnungen in der Lanze oder auch über eine Öffnung am Schließelement erfolgen.

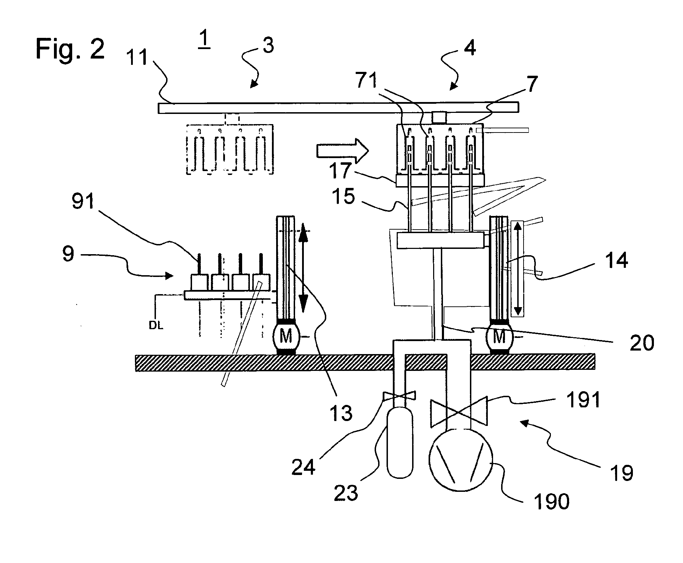

Fig. 2 zeigt die Vorrichtung 1 mit geschlossenem Reaktor. Das Schließelement 17 ist hier mittels der Hubvorrichtung 14 mit der Behälteraufnahme-Vorrichtung 7 zusammengeführt. Nun können die Spritzenkörper innenseitig evakuiert, Prozessgas eingefüllt und ein Niederdruck-Plasma in den Spritzenkörpern gezündet werden. Die Lanze 15 dient dazu als innere Elektrode, die Behälteraufnahmevorrichtung 7 bildet die äußere Elektrode.

Das Einfüllen von Gas, insbesondere auch dem verwendeten Prozessgas und das Abpumpen können auch gleichzeitig erfolgen. Dies bietet den Vorteil, dass das Restgas durch Spülverfahren sehr schnell entfernt wird. Als Spüldauer (Pumpen mit simultanem Einleiten von Gas) wird vorzugsweise eine Dauer von kleiner 60 Sekunden, vorzugsweise höchstens 10 Sekunden, besonders bevorzugt höchstens 5s eingehalten.

Die innere Elektrode ist bei der in den Fig. 1 und 2 gezeigten Bauform in direktem Kontakt mit dem Plasma. Für solche Elektroden in Kontakt mit der Plasmazone wird vorzugsweise allgemein ein korrosionsbeständige Metall, vorzugsweise Aluminium oder einer Aluminium-haltige Legierung verwendet.

Zumindest eine der Elektroden ist an eine Hochfrequenz-Wechselspannungsquelle angeschlossen. Ist nur eine der Elektroden angeschlossen, wird die andere Elektrode auf ein Bezugspotential, beispielsweise Erdpotential gelegt. Durch die angelegte Hochfrequenz-Wechselspannung bildet sich ein elektromagnetisches Feld zwischen den Elektroden, folglich auch im evakuierten und mit Prozessgas unter niedrigem Druck gefüllten Inneren des Spritzenkörpers aus. Das Feld bewirkt dann eine Erzeugung eines Plasmas im Inneren des Spritzenkörpers. Vorzugsweise werden die Spannungen und Drücke so gewählt, dass sich eine Niederdruck-Glimmentladung ausbildet.

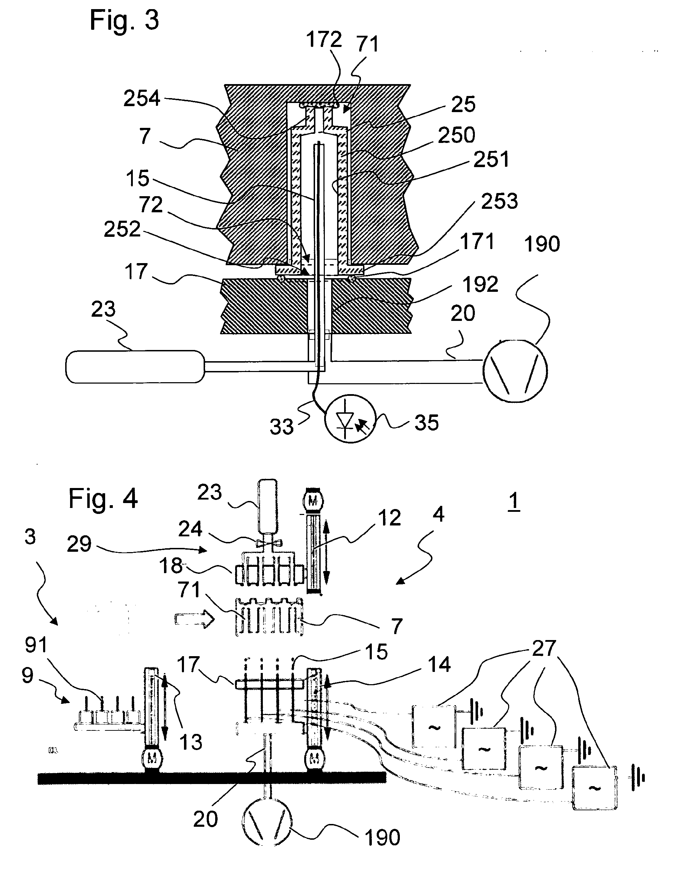

Fig. 3 zeigt im schematischen Querschnitt Details der Vorrichtung 1. Im Speziellen ist in Fig. 3 ein Ausschnitt der an der Behandlungsstation 4 für die Plasmabehandlung mit dem Schließelement 17 zusammengeführten Behälteraufnahme-Vorrichtung 7 gezeigt.

In der Behälteraufnahme-Kammer 71 ist ein Spritzenkörper 25 so eingesetzt und fixiert, dass dessen Öffnung, im Speziellen dessen Kolbenöffnung 252 zum offenen Ende 72 der Behälteraufnahme-Kammer 71 weist. Die Kolbenöffnung am Ende des Spritzenzylinders 250 ist von einem Flansch 253 oder einer Fingerauflage umgeben.

Das Schließelement 7 weist ein Dichtelement 171, hier speziell in Form eines Dichtungsringes auf. Werden, wie in Fig. 3 gezeigt, die Behälteraufnahme-Vorrichtung 7 mit dem Schließelement 17 mittels der Hubvorrichtung zusammengeführt, wird der Spritzenkörper an dessen Kolbenöffnung 252 mit dem Dichtelement 171 abgedichtet. Ein weiteres Dichtelement 172 ist in der Behälteraufnahmekammer 71 vorgesehen, welches die Düse 254 des Spritzenkörpers, vielfach bei Spritzen auch als Luer-Konus bezeichnet, abdichtet. Das den Luer-Konus abdichtende Dichtelement 172 kann gemäß einer vorteilhaften Ausgestaltung der Erfindung auch so ausgestaltet sein, dass es den Spritzen- oder Karpulenkörper gleichzeitig klemmt, so dass der Spritzen- oder Karpulenkörper in der Behälteraufnahmekammer 71 gehaltert wird. Geeignet ist dazu ein Dichtelement 172 in Form eines Dichtringes oder einer Dichtlippe, welche seitlich unter elastischer Verformung beim Einführen des Spritzen- oder Karpulenkörpers am Luer-Konus oder allgemein am der Kolbenöffnung gegenüberliegenden Ende des Spritzen- oder Karpulenkörpers anliegt.

Generell, ohne Beschränkung auf das gezeigte Ausführungsbeispiel sind demgemäß in bevorzugter Weiterbildung der Erfindung zumindest zwei Dichtelemente oder Abdichtungsstellen pro Behälteraufnahmekammer vorgesehen.

Mit den Dichtelementen 171, 172 kann eine vakuumdichte Abdichtung des Spritzenkörpers 25 mit einer gegenüber Atmosphärendruck vakuumdichte Abdichtung mit einer Leckrate unterhalb von 5x10¹ mbar l/s erzielt werden. Der Spritzenkörper 25 ist damit beidseitig abgedichtet und kann durch die Absaugöffnung 192 mittels der Vakuumpumpe 190 evakuiert werden.

Bei der in Fig. 3 gezeigten Ausführungsform ist die Lanze 15 zum Einfüllen und Verteilen des Prozessgases durch die Absaugöffnung 192 hindurch geführt. Selbstverständlich sind aber auch andere Konfigurationen möglich, beispielsweise mit einer oder mehreren neben der Lanze 15 angeordneten Absaugöffnungen 192 oder, wie oben erwähnt mittels einer Lanze, die sowohl Einlassöffnungen für das Prozessgas, als auch Absaugöffnungen aufweist.

Der evakuierte Bereich, also das Innere des Spritzenkörpers 25 definiert dann auch den Bereich, in dem sich das Plasma ausbilden kann. Diese Plasmazone wird durch die Innenseite 251 des Spritzenkörpers begrenzt. Der im Betrieb der Vorrichtung aufgebrachte organische Film auf der Innenseite 251, der mit dem Plasma verfestigt, insbesondere vernetzt wird, ist in Fig. 3 der Einfachheit halber nicht dargestellt.

Wie in Fig. 3 beispielhaft gezeigt, kann ein Lichtleiter 33 vorgesehen werden, welcher Licht aus der Behälteraufnahmekammer 71 zu einem Photodetektor 35 leitet, vorgesehen werden. Mit dieser Anordnung kann das Plasma überwacht werden. In Weiterbildung der Erfindung kann der Plasmabehandlungsprozess auch unter Berücksichtigung der Messwerte des Photodetektors gesteuert werden. Das Faser-Ende des Lichtleiters 33 befindet sich dabei vorzugsweise unmittelbar im Innenraum der Reaktorkammer. Vorzugsweise wird die Faser durch die Elektrodenanordnung, beispielsweise wie in Fig.3 gezeigt, durch die Lanze 15 geführt, so dass die Faser direkt in den Spritzenkörper 25 eingeführt wird und das Plasma-Licht direkt auskoppelt. Wenn demgegenüber eine Photodetektion durch den Behälter hindurch erfolgt, werden durch das Glas- oder KunststoffMaterial bestimmte Frequenzbereiche gegebenenfalls nicht transmittiert oder zumindest, abhängig vom Material, teilweise absorbiert, wodurch eine Lichtdetektion erschwert wird. Die Anordnung mit dem Faserende innerhalb des Behälters liefert diese Messwerte, die nicht durch das Behältermaterial beeinflusst werden. Um eine möglichst breitbandige Erfassung des Plasmalichtes zu ermöglichen, wird vorzugsweise ein Lichtleiter verwendet, der durchlässig für Infrarot-Strahlung, sichtbares Licht und UV-Licht ist.

Gemäß noch einer Ausführungsform der Erfindung wird die Lanze 15 zum Evakuieren verwendet und das Prozessgas über die Düse 254 des Spritzenkörpers eingelassen. Ein Ausführungsbeispiel zeigt Fig. 4 Allgemein, ohne Beschränkung auf das speziell abgebildete Ausführungsbeispiel umfasst die Behandlungsstation 4 ein weiteres Schließelement 18, welches gegenüberliegend zu dem Schließelement 17 mit der Behälteraufnahme-Vorrichtung zusammengeführt wird und die mit den Spritzenkörpern gebildeten Plasmakammern der Plasmabehandlungsstation 4 abdichtet. In Weiterbildung dieser Ausführungsform der Erfindung kann am weiteren Schließelement evakuiert, oder wie dargestellt, Prozessgas zugeführt werden. Bei dem in Fig. 4 gezeigten Ausführungsbeispiel ist das weitere Schließelement 18 demgemäß Bestandteil eines Gasverteilers 29.

Das weitere Schließelement 18 wird gemäß noch einer Weiterbildung der Erfindung wie dargestellt mittels einer weiteren Hubvorrichtung 12 mit der Behälteraufnahme-Vorrichtung 7 zusammengeführt. Alternativ ist es auch möglich, nur eine Hubvorrichtung an einem der Schließelemente 17, 18 vorzusehen, wobei die Hubbewegung nach dem Zusammenführen dieses Schließelements mit der Behälteraufnahme-Vorrichtung 7 die Behälteraufnahme-Vorrichtung 7 mitnimmt und mit dem weiteren Schließelement zusammenführt. Für die Gaszuführung können bei zumindest teilweise stationärem Komponenten des Gasverteilers 19 wie oben bereits erwähnt, ein oder mehrere flexible Schläuche für die Gaszuführung zum Schließelement 18 verwendet werden.

Generell, ohne Beschränkung auf die spezielle Ausgestaltung der in den Figuren gezeigten Ausführungsbeispiele ist die Anordnung aus Behälteraufnahme-Vorrichtung 7 und Transporteinrichtung vorzugsweise in vertikaler Richtung im Wesentlichen unbeweglich. Dies bedeutet, dass die Anordnung in vertikaler Richtung vorzugsweise vollständig unbeweglich ist oder die Bewegung, beispielsweise bei der vorstehend erwähnten Mitnahme der Behälteraufnahme-Vorrichtung 7 weniger als 10% der Hubbewegung beträgt.

Bei dem in Fig. 4 gezeigten Beispiel sind mehrere Wechselspannungsgeneratoren 27 vorgesehen, um die für die Erzeugung des Plasmas erforderliche Energie bereitzustellen. Dabei ist für jede der Behälteraufnahme-Kammern 71 ein separater Wechselspannungsgenerator 27 vorgesehen. Wie in Fig. 4 dargestellt, sind die Wechselspannungsgeneratoren 27 an die Lanzen 15 angeschlossen. Die Behälteraufnahme-Vorrichtung 7 wird für die Plasmabehandlung geerdet, wobei auch die Wechselspannung der Wechselspannungsgeneratoren 27 auf Erdpotential bezogen ist. Damit bilden die Lanzen 15 eine erste Elektrode und die Innenwandung der Behälteraufnahme-Kammern 71 die zweite Elektrode.

Allgemein ist es günstig, wie auch in dem in Fig. 4 gezeigten Beispiel für die Elektroden jeder Behälteraufnahmekammern jeweils eine separat ansteuerbare Hochfrequenz-Energiequelle oder Wechselspannungsquelle vorzusehen, vorzugsweise einer Hochspannungsquelle mit einer Frequenz im Mittelfrequenz-Bereich von 0,1 - 200kHz. Dies ist von Vorteil, da für jede Plasma-Kammer die eingekoppelte Energie, beispielsweise über den Plasma-Strom, separat so eingestellt werden kann, dass in allen Plasma-Kammern eine gleich hohe Plasma-Intensität vorliegt.

Alternativ kann auch eine Anregung des Niederdruck-Plasmas mit Mikrowellen, vorzugsweise im Bereich um 2,45 GHz oder mit Radiofrequenz im Bereich um 13,56 MHz erfolgen.

Um eine gleichmäßige Glimmentladung zu erzeugen, welche auch zu einer tiefgehenden Vernetzung des organischen Films führt, haben sich weiterhin allgemein, ohne Beschränkung auf die in den Figuren dargestellten Ausführungsbeispiele folgende Parameter für die Erzeugung des Plasmas als günstig erwiesen:

Die an den Elektroden angelegte mittelfrequente Spannung liegt vorzugsweise im Bereich von 0,5kV - 10kV, besonders bevorzugt im Bereich von 0,8kV - 3 kV.

Für die Plasma-Entladung in einem Behälter wird vorzugsweise ein Strom im Bereich von 1mA bis etwa 200mA eingestellt.

Erfindungsgemäß kann auf überraschend einfache Weise sicher gestellt werden, dass durch eine bevorzugte Ausführungsform der Vorrichtung eine homogene Oberflächen-Behandlung insbesondere bei zylinder-symmetrischen Behältern ermöglicht wird:

Die Vorrichtung umfasst dabei wenigstens eine Zentriervorrichtung, durch die die Behälteraufnahme-Vorrichtung bzw. die Transporteinrichtung zentriert werden kann. Mit anderen Worten ist eine Zentriervorrichtung vorgesehen, mit welcher die Behälteraufnahme-Vorrichtung zu den Behandlungswerkzeugen an den Behandlungsstationen in der Ebene senkrecht zur Hubrichtung der Behandlungswerkzeuge zentrierbar ist.

Ferner umfasst die Erfindung in einer bevorzugten Ausführungsform ein Verfahren, bei dem die Behandlungswerkzeuge axial-symmetrisch zu den Behältern positioniert bzw. während der Oberflächenbehandlung axialsymmetrisch geführt werden. Beispielsweise werden die Innen-Elektroden axialsymmetrisch in die Behälter eingeführt und insbesondere auch axialsymmetrisch positioniert. In einem weiteren Beispiel werden die Sprühdüsen werden axialsymmetrisch positioniert und während der Oberflächenbehandlung axialsymmetrisch, vorzugsweise mit konstanter Geschwindigkeit in Relation zu den Behältern, geführt.

Allgemein ist es weiterhin günstig, sowohl für einen homogenen Feldaufbau, als auch eine homogene Verteilung des Prozessgases, die Elektroden bzw. Lanzen 15 mittels einer geeigneten Einrichtung, etwa einer Zentrier-, beziehungsweise Positioniereinrichtung zu zentrieren, so dass die Lanzen 15 entlang der Mittenachse der Spritzenzylinder 250 verlaufen. Gleiches gilt auch für die Sprühdüsen. Auch diese werden vorzugsweise auf die Mittenachse der Spritzenkörper oder allgemeiner, der zu beschichtenden Behälter zentriert, um eine gleichmäßige Schichtdicke des organischen Films zu erhalten.
Die Zentrierung kann beispielsweise durch wenigstens eine justierbare Klemmvorrichtung erfolgen, durch die die Elektroden bzw. die Sprühdüsen axialsymmetrisch zu den in der Behälteraufnahmeeinrichtung angeordneten Behältern zentriert werden. In Weiterbildung der Erfindung kann wenigstens eine justierbare Klemmvorrichtung als Bestandteil der Zentriervorrichtung vorgesehen sein, durch die die Transporteinrichtung mit der Behälteraufnahme-Vorrichtung in Relation zu den Behandlungswerkzeugen axialsymmetrisch zentriert wird.

Beispielsweise können die Justierschrauben der Klemmvorrichtung nach der Positionierung versiegelt werden, um sicherzustellen, dass sich die Positionierung im Betrieb nicht verstellt. In einer anderen Ausführungsform kann aber auch wenigstens ein selbst-zentrierendes Bauteil verwendet werden, das geeignet positioniert wird.

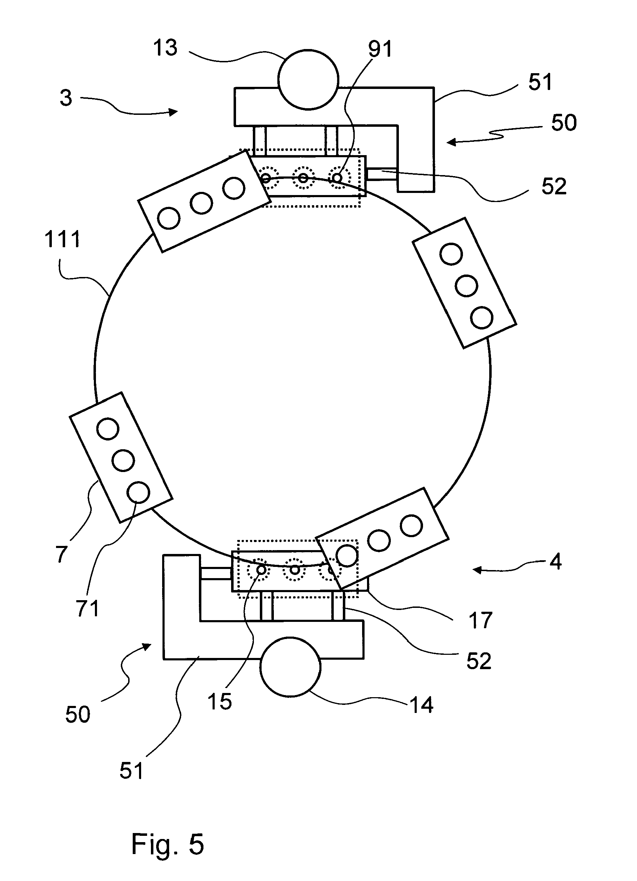

Ein Ausführungsbeispiel einer erfindungsgemäßen Vorrichtung 1 zur Plasmabehandlung mit Zentriervorrichtungen zeigt Fig. 5. Die Vorrichtung 1 ist hier in Aufsicht dargestellt. Die Transporteinrichtung 11 ist als Karussell oder Runddrehtisch 111 ausgebildet. Die Behälteraufnahmevorrichtungen 7 sind auf dem Runddrehtisch 111 angeordnet und werden mit diesem entlang eines kreisförmigen Pfads zu den Behandlungsstationen 3, 4 befördert. Jede der Behandlungsstationen 3, 4 weist bei diesem Ausführungsbeispiel eine Zentriervorrichtung 50 auf. Die Zentriervorrichtungen 50 weisen jeweils eine Halterung 51 auf, welche an der jeweiligen Hubeinrichtung 13, beziehungsweise 14 befestigt ist. Die Behandlungswerkzeuge, im Speziellen hier die Sprühdüsen 91 und das Plasmabehandlungs-Werkzeug mit Schließelement 17 und Lanzen 15 sind über Justierschrauben 52 mit der Halterung verbunden. Die Justierschrauben 52 sind so angeordnet, dass die Behandlungswerkeuge, in der Ebene senkrecht zur Hubrichtung der Hubeinrichtungen 13, 14 bewegbar sind. Die Justierschrauben 52 werden so eingestellt, dass die jeweiligen Behandlungswerkeuge, im Speziellen die Lanzen 15 und Sprühdüsen 91 an den vorgesehenen Haltepositionen der Behälteraufnahmevorrichtungen 7 (diese sind in gestrichelten Linien eingezeichnet), axialsymmetrisch zu den Längsachsen der in den Behälteraufnahmekammern 71 angeordneten Behältern angeordnet sind. Mittels geeigneter Klemmvorrichtungen werden die Justierschrauben 52 fixiert. Alternativ oder zusätzlich kann auch die Drehachse 112 des Runddrehtisches 111 in Richtung senkrecht zur Hubrichtung mittels einer entsprechenden Zentriervorrichtung einjustiert werden.

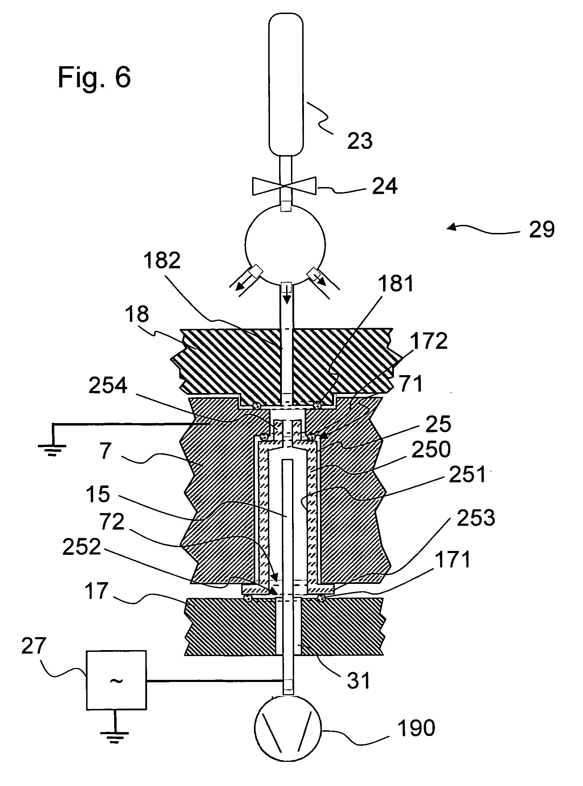

Fig. 6 zeigt einen Ausschnitt der Vorrichtung 1 mit der Behälteraufnahmevorrichtung 7 in der Behandlungsstation 4, wobei zur Bildung eines Plasmareaktors die Schließelemente 17, 18 mit der Behälteraufnahmevorrichtung 7 zusammengeführt sind.

Zur Bildung einer gasdichten Verbindung ist zwischen dem oberen Schließelement 18 des Gasverteilers 29 und der Behälteraufnahme-Vorrichtung 7 ein Dichtelement 181 vorgesehen. Dieses dichtet den mit der Spritzendüse 254 kommunizierenden Gaseinlasskanal 182 des Schließelements 18 gegenüber dem atmosphärischen Umgebungsdruck ab.

Die Lanze 15, an welcher ein Wechselspannungsgenerator 27 angeschlossen ist, ist mittels eines Isolationselements vom Schließelement 17 elektrisch isoliert. Die Behälteraufnahme-Vorrichtung, oder zumindest die Innenwandung der Behälteraufnahme-Kammer 71 werden auf Massepotential gelegt, welches auch das Bezugspotential des Wechselspannungsgenerators 27 ist, so dass sich zwischen der Innenwandung der Behälteraufnahme-Kammer 71 und der Lanze 15 aufgrund der angelegten Wechselspannung ein elektrisches Wechselfeld oder elektromagnetisches Feld ausbildet.

Gemäß einer bevorzugten Ausgestaltung der Erfindung wird ein gepulstes Plasma erzeugt. Dieses kann durch Einsatz einer gepulsten Wechselspannung erfolgen. Die Pulsfrequenz ist dabei vorzugsweise mindestens 10-mal kleiner als die Wechselspannungs-Frequenz. In einer besonders bevorzugten Ausführungsform kann das gepulste Plasma aber auch durch Anlegen einer kontinuierlichen Wechselspannung erzeugt werden. Dabei zeigte sich auf überraschend einfache Weise, dass durch Positionierung eines dielektrischen Körpers, besonders einfach in Form des Substrats selbst, zwischen den beiden Elektroden eine gepulste Plasmazone innerhalb des Behälters erzeugt werden kann.

Allgemein sind folgende Prozessparameter besonders günstig für gute Haft- und Oberflächeneigenschaften:

Die Oberflächenbehandlung der organischen Schicht erfolgt mit einem gepulsten Plasma mit einem Tastverhältnis T = Pₒₙ/(P_{off}+Pₒₙ) < 0,5. Mit anderen Worten ist die Dauer des Pulses weniger als halb so lang wie die Dauer einer Periode des Pulszyklus aus Puls und Pulspause. Daraus ergibt sich, dass die Pulsdauern kürzer sind, als die Pulspausen. Besonders bevorzugt wird ein Tastverhältnis T = Pₒₙ/ (P_{off}+Pₒₙ) < 0, 3. Dieses Tastverhältnis ist vorteilhaft für einen kontrollierten, kleinen Energieeintrag zur effizienten Vernetzung der Schicht, einer Verhinderung einer zu starken Aushärtung oder sogar einer Überhitzung der Gleitschicht und des Behälters, sowie einer längeren Gaslaufzeit für den Abtransport von flüchtigen Verbindungen zwischen den Pulsen, um Verunreinigungen der Schicht zu minimieren.

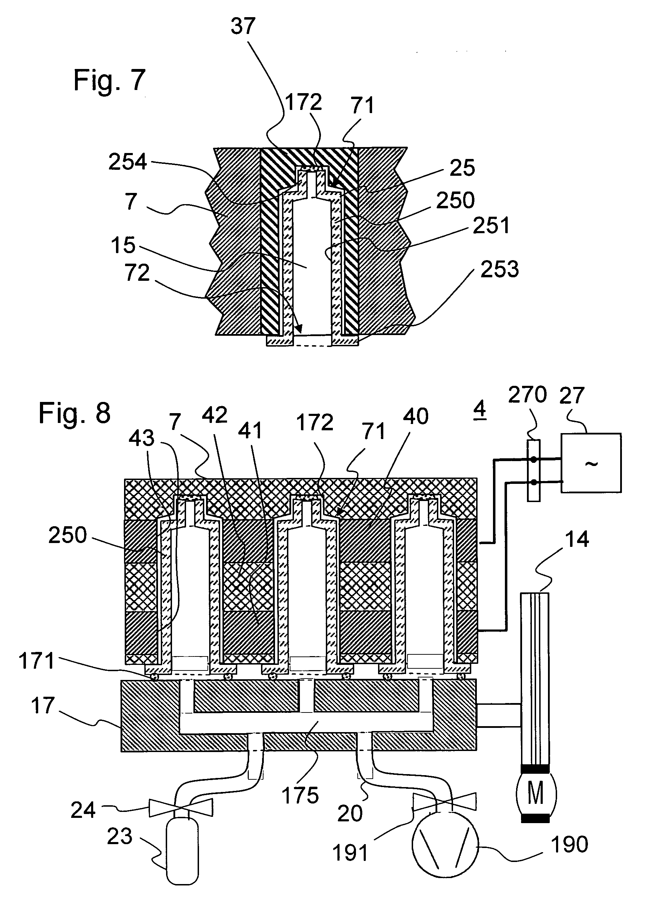

Fig. 7 zeigt einen Ausschnitt der Behälteraufnahme-Vorrichtung 7 gemäß noch einer mit allen Ausführungsformen der Erfindung kombinierbaren Weiterbildung der Erfindung. Das spezielle, in Fig. 7 gezeigte Ausführungsbeispiel geht von der in Fig. 3 dargestellten Anordnung aus. Bei dieser Ausführungsform der Erfindung werden die Behälteraufnahmekammern 71 nicht direkt im Körper der Behälteraufnahme-Vorrichtung 7 ausgebildet. Vielmehr sind die Behälteraufnahmekammern 71 zumindest teilweise durch die Innenflächen von Behälteraufnahme-Hülsen 37 ausgebildet, die wiederum in passende Aufnahmen 38 in der Behälteraufnahme-Vorrichtung 7 eingesetzt werden. Dies bietet den Vorteil, die Behälteraufnahme-Vorrichtung 7 für verschiedene Größen von zu behandelnden Behältern, wie etwa für Spritzen unterschiedlichen Füllvolumens verwenden zu können. Wie in Fig. 7 weiter gezeigt, kann die Innenseite der Behälteraufnahme-Hülse 37 auch auf die verschiedenen Außendurchmesser der Behälter, hier auf die Außendurchmesser von Spritzenzylinder 250 und Düse 254 angepasst werden, um größere Abstände zur Außenoberfläche der Behälter zu vermeiden. Mittels der Behälteraufnahme-Hülse 37 wird auch der zu behandelnde Behälter auf eine vorgesehene Position zentriert, so dass die Behandlungswerkzeuge, insbesondere die Sprühdüse, und/oder eine Innenelektrode und/oder eine Gaslanze konzentrisch zur Behälter-Längsachse eingeführt werden.

Gemäß noch einer Weiterbildung der Erfindung wird die Innenoberfläche der Behälteraufnahme-Kammer 71 zumindest teilweise durch ein Dielektrikum gebildet. Vorzugsweise liegt dessen Dielekrizitätszahl im Bereich von 1,2 bis 80. Im einfachsten Fall wird dazu eine Behälteraufnahme-Hülse 37 aus dielektrischem Material oder mit einer Innenauskleidung aus dielektrischem Material verwendet. Besonders bevorzugt wird ein Kunststoff als Dielektrikum verwendet. Überraschend ist die Zündung eines Plasmas auch ohne weiteres mit diesem zusätzlich zum typischerweise dielektrischen Behälter zwischen den Elektroden vorhandenen Dielektrikum möglich.

Fig. 8 zeigt beispielhaft Teile der Vorrichtung 1 gemäß noch einer Ausführungsform der Erfindung. Im Speziellen ist die mit Spritzenkörpern 25 bestückte Behälteraufnahme-Vorrichtung 7 in der Plasmabehandlungsstation 4 gezeigt, wobei das Schließelement zur Bildung eines Plasmareaktors mit der Behälteraufnahme-Vorrichtung 7 zusammengeführt ist, so dass die Spritzenkörper 25 an deren Kolbenöffnung mittels der Dichtelemente 171 abgedichtet werden.

Diese in Fig. 8 beispielhaft dargestellte Ausführungsform der Erfindung basiert darauf, dass das elektromagnetische Feld in der Plasmazone im Inneren der Behälter durch zumindest zwei außerhalb des Behälters angeordnete Elektroden erzeugt wird. Im Speziellen können, wie bei der in Fig. 8 gezeigten Ausführungsform die Elektroden 40, 41 axial entlang der Behälterachse, beziehungsweise axial versetzt in unterschiedlichem Abstand zur Behälteröffnung angeordnet sein. Diese Ausführungsform der Erfindung bietet den Vorteil, dass das Feld ohne eine Innenelektrode erzeugt wird. Dies ermöglicht, ein Feld in Bereichen zu erzeugen, die ansonsten mittels einer Innenelektrode nur schwer zugänglich sind. Bei der Plasmabehandlung von Spritzenkörpern 25 kann auf diese Weise auch die Innenseite des Luer-Konus, beziehungsweise der Düse 254 des Spritzenkörpers 25 behandelt werden.

Vorteilhaft können hier, wie auch bei dem in Fig. 8 gezeigten Beispiel, die Elektroden 40, 41 gemeinsam das Feld in mehreren Behälteraufnahmekammern 71 erzeugen. Dazu sind die beiden Elektroden 40, 41 über eine elektrische Kopplungseinrichtung 270 an eine gemeinsame Spannungsversorgung in Form eines WechselspannungsGenerators 27 angeschlossen. Vorzugsweise ist zumindest eine, besonders bevorzugt sind, wie in Fig. 8 dargestellt, beide Elektroden 40, 41 Bestandteil der Behälteraufnahme-Vorrichtung 7.

Die Elektroden 40, 41 sind durch einen Isolator 42 axial voneinander getrennt angeordnet und weisen Öffnungen 43 auf, welche die Behälteraufnahmekammern 71 umschließen.

Ohne Beschränkung auf die nachfolgend anhand der weiteren Figuren beschriebenen Ausführungsbeispiele können in Weiterbildung der Erfindung weitere Behandlungsstationen vorgesehen werden. So kann gemäß noch einer Weiterbildung der Erfindung allgemein eine zweischichtige Innenbeschichtung der Behälter vorgesehen werden. Dabei kann die zuerst aufgetragene Schicht die Haftung einer darüber liegenden, also entsprechend danach aufgetragenen Gleitschicht verbessern. Die erfindungsgemäße Vorrichtung kann dazu eine erste Behandlungsstation eine Vorrichtung zum Aufbringen eines ersten organischen Films, vorzugsweise durch Aufsprühen, eine in Transportrichtung der ersten Behandlungsstation nachgeordnete zweite Behandlungsstation mit einer Einrichtung zur Erzeugung eines Niederdruck-Plasmas im Inneren der Behälter, eine dritte Behandlungsstation zum Aufbringen eines weiteren organischen Films, wobei die dritte Behandlungsstation der zweiten Behandlungsstation in Transportrichtung nachgeordnet ist, und eine vierte, der dritten Behandlungsstation in Transportrichtung nachgeordnete Behandlungsstation mit einer weiteren Einrichtung zur Erzeugung eines Niederdruck-Plasmas im Inneren der Behälter umfassen.

Als weitere Behandlungsstation kann gemäß noch einer Weiterbildung der Erfindung allgemein eine Behandlungsstation zum Trocknen des aufgebrachten organischen Films vor der Plasmabehandlung vorgesehen werden. Die Trocknung kann ebenso wie das Aufbringen des organischen Films und die Plasmabehandlung mit Werkzeugen oder Vorrichtungselementen bewerkstelligt werden, welche mittels einer Hubvorrichtung mit der Behälteraufnahme-Vorrichtung 7 zusammengeführt werden. So kann zur Trocknung des organischen Films eine Lanze in die Behälter eingeführt werden, mit welcher das Behälterinnere mit einem Gas, vorzugsweise Druckluft gespült wird. Durch das Spülen werden die bei der Trocknung ausgasenden Stoffe, wie beispielsweise Lösungsmittel abgeführt. Alternativ oder zusätzlich kann eine Heizeinrichtung eingeführt werden, um die Trocknung des organischen Films zu beschleunigen.

Eine zweischichtige Beschichtung, bei der wenigstens eine der Beschichtungen, vorzugsweise beide organischen Schichten mittels des jeweils einwirkenden Niederdruck-Plasmas vernetzt sind und wie sie mit der vorstehend beschriebenen Vorrichtung herstellbar ist, eignet sich besonders als Gleitfilm auf Pharma-Packmitteln aus Glas. Gemäß einer Weiterbildung der Erfindung weist die Vorrichtung dazu eine weitere Behandlungsstation zur Vorbehandlung in Form einer Sprühstation auf, mit der eine Zwischenschicht innenseitig auf dem Behälter aufgebracht wird. Nach dem Aufbringen der Zwischenschicht erfolgt ein Trocknungs- und/oder Aushärte-Schritt, bei dem flüchtige Komponenten der Zwischenschicht zumindest weitgehend aus dem Behälter-Innenraum entfernt werden, vorzugsweise durch einen nachfolgenden Behandlungsschritt in Form einer thermischen Behandlung, eines Sprühprozesses mit Druckluft oder in Form eines Evakuier-Prozesses.

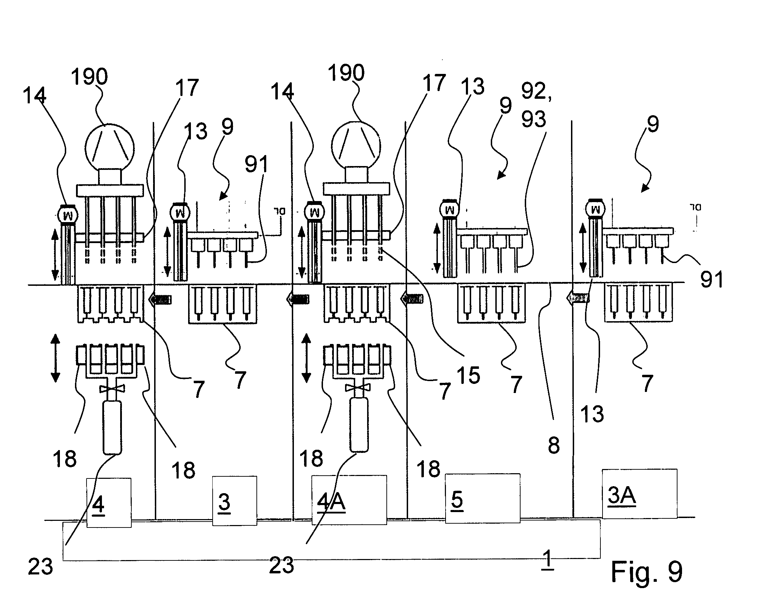

Ein Beispiel dazu zeigt Fig. 9. Insgesamt weist die Vorrichtung 1 fünf Behandlungsstationen auf. Die beiden letzten Behandlungsstationen 3, 4 entsprechen den zuvor anhand der vorhergehend beschriebenen Behandlungsstationen 3, 4 zum Aufbringen eines organischen, insbesondere silikonfreien Films (Behandlungsstation 3) und Vernetzen des Films im Niederdruck-Plasma (Behandlungsstation 4).

Vor dem Aufbringen der organischen Gleitschicht wird aber in den Behandlungsstationen 3A, 5 und 4A eine Zwischenschicht aufgebracht. Diese dient dazu, eine feste Haftung der Gleitschicht auf der Behälterinnenseite zu gewährleisten. Dabei wird in der Behandlungsstation 3A ein Film mit Sprühdüsen 91 aufgesprüht und in der Behandlungsstation 4A mit einem Niederdruck-Plasma behandelt. Den Behandlungsstationen 3A, 4A ist eine weitere Behandlungsstation 5 zwischengeschaltet. Auch diese Behandlungsstation umfasst Behandlungswerkzeuge 9, welche mittels einer Hubvorrichtung 13 mit der Behälteraufnahme-Vorrichtung 7 zusammengeführt werden. Als Behandlungswerkzeuge 9 werden hier Druckluft-Lanzen 92 verwendet, mit welchen das Innere der Behälter gespült wird, um den organischen Film zu trocknen. Gemäß einer alternativen oder zusätzlichen Ausführungsform können als Behandlungswerkzeuge 9 hier auch Heizdorne 93 in die Behälter eingeführt werden, um die Schichten zu trocknen oder auszuhärten.

Beispielhaft sind die Plasma-Behandlungsstationen 4A, 4 entsprechend den in Fig. 4 und Fig. 6 dargestellten Ausführungsformen ausgebildet, bei welcher das Prozessgas durch ein weiteres Schließelement 18 gegenüberliegend zum Schließelement 17 zugeführt wird. Ebenso ist es möglich, die Behandlungsstationen 4, 4A entsprechend den in Fig. 1, 2 und 3 dargestellten Ausführungsformen auszubilden.

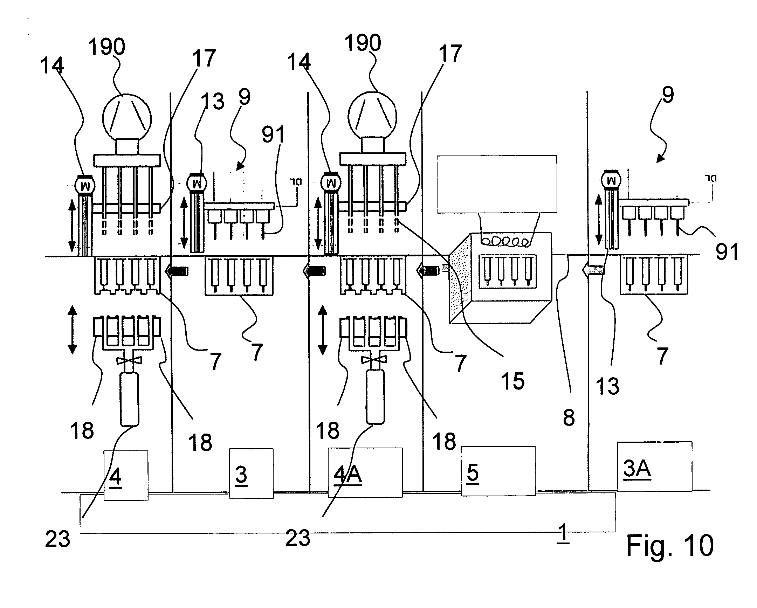

In Fig. 10 ist eine Variante des in Fig. 9 gezeigten Ausführungsbeispiels gezeigt. Anstelle einer Trocknung des organischen Materials der Zwischenschicht durch in die Spritzenkörper mittels einer Hubeinrichtung 13 eingeführte Druckluftlanzen 92 erfolgt die Trocknung in einem Durchlaufofen 45. Hierbei wird die mit den Behältern, beziehungsweise hier speziell den Spritzenkörpern bestückte Behälteraufnahme-Vorrichtung 7 durch die Transporteinrichtung durch den Durchlaufofen 45 geführt.

Nachfolgend werden Ausführungsbeispiele für das mit einer wie vorstehend beschriebenen Vorrichtung 1 durchführbare Verfahren angegeben.

### Ausführungsbeispiel 1:

In eine Behälteraufnahme-Vorrichtung 7 entsprechend der in Fig. 4 und 5 gezeigten Ausführungsform mit vier Behälteraufnahmekammern 71 werden vier gewaschene und getrocknete Glas-Spritzenkörper 25 aus Borosilikatglas (Typ Fiolax klar), Format 1,25ml eingebracht und über eine Haltervorrichtung fixiert. Dabei wird von jeder Spritze der Luer-Konus jeweils über ein Dichtelement 172 im Bereich der oberen Öffnungen gegenüber Atmosphärendruck abgedichtet. Die Behälteraufnahme-Vorrichtung 7 wird in die Vorrichtung 1 eingesetzt und mittels der Transporteinrichtung horizontal zur Behandlungsstation 3 befördert. Dabei sind die Spritzenkörper 25 mit dem Luer-Konus, beziehungsweise der Düse 254 in vertikaler Richtung nach oben (also in Richtung 12 Uhr) orientiert.

Die Behälteraufnahme-Vorrichtung 7 wird auf eine Halteposition gebracht und dabei so positioniert, dass die Mittelpunktsachsen der Spritzenkörper 25 zentral zur den verlängerten Achsen der als Zweistoffdüsen ausgebildeten Sprühdüsen 91 orientiert sind. Über die pneumatische Hubvorrichtung 13 werden die vier Zweistoffdüsen gleichzeitig in vertikaler Richtung mit der Behälteraufnahme-Vorrichtung 7 zusammengeführt und dabei in die Spritzenkörper innenseitig eingeführt.

In einem dynamischen Prozess, bei dem die Sprühdüsen 91 in die Spritzenkörper 25 hineinfahren, wird mittels einer Zweiphasen-Strömung aus Stickstoff und einem Fluid aus Perfluorpolyether, Typ Fomblin M100, die Innenoberfläche des Spritzen-Zylinders 250 mit einer Silikon-freien organischen Schicht beschichtet. Der Sprühprozess erfolgt jeweils innerhalb einer Dauer von 1,4s, bei einem Massenfluss an Gas von 84,5mg/s und es wird dabei eine flächenbezogene Stoffmenge von 0,07 Mikroliter pro Quadratzentimeter auf der Innenoberfläche der jeweiligen Spritzenkörper 25 aufgebracht. Während des Sprühvorgangs werden die Sprühdüsen mit konstanter Geschwindigkeit aus den Spritzenkörpern 25 herausgefahren.

Sobald der Sprühvorgang beendet ist und die Sprühdüsen auf ihre Ausgangsposition zurück gekehrt sind, wird unmittelbar anschließend die Behälteraufnahme-Vorrichtung 7 mit den mit dem Fluid-Film vorbeschichteten Spritzen innerhalb einer Dauer unterhalb von 5s mit der als Runddrehtisch ausgebildeten Transporteinrichtung 11 zur Behandlungsstation 4 befördert und zur vorgesehenen Halteposition gebracht.

In der Behandlungsstation 4 wird über die Hubvorrichtung 14 das Schließelement 17, an dem vier Hohlelektroden mit Dichtringen befestigt sind, zur Behälteraufnahme-Vorrichtung 7 in vertikaler Richtung geführt. Auf der gegenüberliegenden Seite wird das weitere Schließelement 18, an dem ein symmetrischer Gasverteiler 29 mit vier Gasleitungen angeschlossen ist, über die Hubvorrichtung 12 zur Behälteraufnahme-Vorrichtung 7 geführt. Die Abdichtung erfolgt mit vier weiteren Dichtungselementen 181 in Form von Dichtungsringen, die sich auf der Behälteraufnahme-Vorrichtung 7 befinden.

Nach Zuführen der Schließelemente 17, 18 wird zusammen mit der Behälteraufnahme-Vorrichtung 7 und den Spritzenkörpern 25 eine gegenüber Atmosphärendruck gasdichte Plasma-Reaktor-Kammer gebildet. Ferner wird ein elektrischer Kontakt zwischen den Elektroden der Behälteraufnahme-Vorrichtung 7 und den Wechselspannungs-Generatoren in Form von Hochspannungs-Mittelfrequenzgeneratoren hergestellt.

Anschließend wird mittels einer Vakuumpumpe 190 die Reaktor-Kammer auf einen Basisdruck unterhalb von 0,5mbar evakuiert und es wird nachfolgend über den Gasverteiler 29 reines Argongas in den Plasma-Reaktor eingeleitet. Dabei wird über einen Massenfluss-Regler der gesamte Molekularfluss für alle Reaktorkammern eingeregelt und symmetrisch auf alle vier Reaktor-Kammern aufgeteilt. Über eine Drosselklappe wird ein Prozessdruck mit einem Wert von 5 mbar eingeregelt.

Über die Mittelfrequenzgeneratoren wird anschließend eine elektrische Leistung im Bereich von 140W - 560W (1W = 1VA) bei einer Frequenz von 100kHz jeweils an den einzelnen Elektroden der Plasmakammern separat eingespeist, so dass in den Plasma-Kammern eine Niederdruck-Glimmentladung zündet und aufrecht erhalten wird. Die simultane Plasma-Behandlung der fluiden Filme auf den Oberflächen der Spritzenkörper 25 erfolgt für eine Dauer von 5 Sekunden. Innerhalb dieser Dauer werden die silikonfreien Gleitfilme auf den Spritzenoberflächen simultan ausgehärtet.

Nachfolgend wird die Reaktor-Kammer mit Stickstoffgas geflutet. Die Behälteraufnahme-Vorrichtung 7 wird aus der Behandlungsstation 4 ausgeschleust und die Spritzenkörper 25 werden aus der Behälteraufnahme-Vorrichtung 7 entnommen.

An den mit Silikon-freier Gleitschicht beschichteten Glasspritzen werden folgende Tests durchgeführt:

Unmittelbar nach Herstellung der Gleitschichten werden in einen ersten Teil der Spritzen Silikon-freie Stopfen vom Typ FM257 von der Fa. Helvoet eingesetzt und es wird die Haft- und Gleitreibung bestimmt.

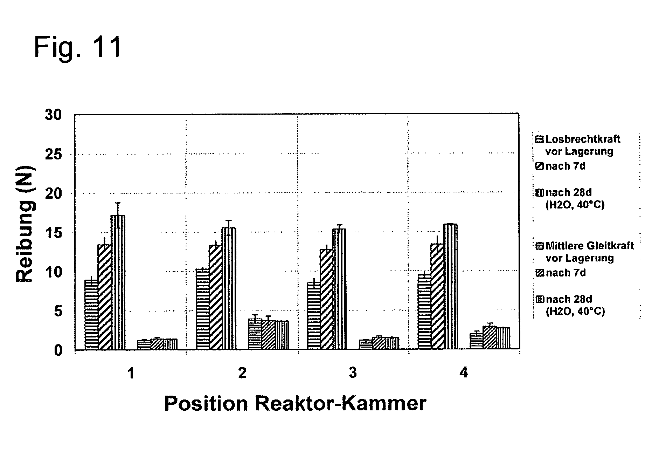

Ein weiterer Teil der Silikon-freien Glasspritzen wird mit Wasser befüllt, mit einer Verschlusskappe für die Düse, auch als "Tip Cap" bezeichnet, und nachfolgend mit gleichem Stopfen verschlossen und bei einer Temperatur von 40°C für Dauern von 7 Tagen und 28 Tagen eingelagert. Nachfolgend wird auch von diesen mit Wasser befüllten Spritzen die Haft- und Gleitreibung nach Einlagerung gemessen. Dabei sind die Spritzen so nummeriert, dass diese den einzelnen Positionen der Sprüh- und Plasma-Behandlungsanlage zugeordnet werden können. Die Messwerte der Haft- und Gleitreibungsmessungen ist in Fig. 11 dargestellt.

Die Losbrechkräfte liegen für alle auf den Positionen 1-4 hergestellten Gleitschichten vor Lagerung im Bereich von 9N - 10N und zeigen einen für Gleitschichten typischen Anstieg nach Einlagerung. Nach 28 Tagen Einlagerung bei 40°C liegt die Losbrechkraft im Bereich von 15N - 17N. Die Gleitreibungswerte der Spritzen liegen für alle Reaktor-Kammern im Bereich von 1N - 4N und sind insbesondere während der Lagerdauer stabil.

Die Testergebnisse zeigen, dass mit der erfindungsgemäßen Vorrichtung 1 silikonfreie Gleitschichten in einem aufgrund der simultanen Behandlung mehrerer Spritzenkörper für die Massenfertigung tauglichen Verfahren hergestellt werden können und dabei sogar mit unsilikonisierten Stopfen Losbrech- und Gleitkräfte erzielt werden, die den Anforderungen für die Applikation des pharmazeutischen Primärpackmittels genügen. Insbesondere sind die im erfindungsgemäßen Verfahren auf der Mehrplatz-Vorrichtung hergestellten Gleitschichten im Bereich der Kontaktfläche mit Wasser lagerstabil, was die über die Lagerdauer konstante und sehr niedrige Gleitreibung zeigt.

**Ausführungsbeispiel 2:** In eine Behälteraufnahme-Vorrichtung 7 entsprechend der in Fig. 4 und 5 gezeigten Ausführungsform mit vier Behälteraufnahmekammern 71 werden vier Spritzenkörper 25 aus COC (Cyclo-Olefin-Copolymer), Format 2,25ml, eingebracht und über eine Haltevorrichtung fixiert. Dabei wird von jeder Spritze der Luer-Konus jeweils über ein Dichtelement 172 im Bereich der oberen Öffnungen gegenüber Atmosphärendruck abgedichtet. Die Behälteraufnahme-Vorrichtung 7 wird in die Vorrichtung 1 eingesetzt und horizontal zur Behandlungsstation 3 befördert und auf eine Halteposition gebracht.

Analog zum ersten Beispiel wird ein silikon-freies Fluid aus Perfluorpolyether auf die Innenoberfläche der COC-Spritzenzylinder 25 aufgesprüht. Sobald der Sprühvorgang beendet ist und die Sprühdüsen 91 auf ihre Ausgangsposition zurückgekehrt sind, wird unmittelbar die Behälteraufnahme-Vorrichtung 7 mit den vorbeschichteten Spritzen innerhalb einer Dauer unterhalb von 5s über den Runddrehtisch zur Behandlungsstation 4 befördert und zur vorgesehenen Halteposition gebracht.

In der Behandlungsstation 4 wird über die Hubvorrichtung 141 das Schließelement 17, an dem vier Hohlelektroden mit Dichtringen befestigt sind, zur Behälteraufnahme-Vorrichtung 7 in vertikaler Richtung geführt. Auf der gegenüberliegenden Seite der Behälteraufnahme-Vorrichtung 7 wird das Schließelement 18, an dem der Gasverteiler 29 angeschlossen ist, über die weitere Hubvorrichtung 12 zur Behälteraufnahme-Vorrichtung 7 geführt und über vier weitere Dichtungsringe 172 auf der Behälteraufnahme-Vorrichtung 7 verbunden.

Nach Zusammenführen der Schließelemente 17, 18 mit der Behälteraufnahme-Vorrichtung 7 werden gegenüber Atmosphärendruck gasdichte Plasma-Reaktor-Kammern gebildet. Ferner wird ein elektrischer Kontakt zwischen den Elektroden der Behälteraufnahme-Vorrichtung 7 und den Hochspannungsgeneratoren hergestellt.

Anschließend wird mittels einer Vakuumpumpe die Reaktor-Kammer auf einen Basisdruck unterhalb von 0,5mbar evakuiert und es wird nachfolgend über den Gasverteiler 29 reines Argongas eingeleitet. Dabei werden analoge Parameter für den Plasmaprozess wie in Bsp.1 appliziert, so dass die fluiden Filme auf den COC-Spritzenkörpern 25 simultan durch das Plasma vernetzt werden.

Nachfolgend wird die Reaktor-Kammer mit Stickstoffgas geflutet. Die Behälteraufnahme-Vorrichtung 7 wird aus der Behandlungsstation 4 ausgeschleust und die Spritzenkörper entnommen.

An den mit silikonfreier Gleitschicht beschichteten COC-Spritzen werden analoge Tests zur Haft- und Gleitreibung durchgeführt wie in Ausführungsbeispiel 1. Es zeigt sich, dass auf COC-Spritzenkörpern ebenso gute Haft- und Gleitreibungseigenschaften der Silikon-freien Gleitschichten wie auf Glasspritzen erreicht werden können.

**Ausführungsbeispiel 3:** In eine Behälteraufnahme-Vorrichtung 7 mit vier Behälteraufnahmekammern 71 werden vier Glasspritzen entsprechend dem obigen Ausführungsbeispiel 1, Format 1,25ml eingebracht und über eine Haltevorrichtung fixiert. Die Glasspritzen werden entsprechend der in Fig. 9 gezeigten Vorrichtung in einem mehrstufigen Herstellprozess zweischichtig beschichtet. Analog zum Ausführungsbeispiel 1 werden sehr geringe Reibwerte für die zweischichtig beschichteten Glasspritzenkörper erzielt. Dabei erweist sich als Verfahren für die Trocknung und Anbindung der 1. Schicht sowohl ein Verfahren durch Druckluftlanzen 92, als auch durch Heizdorne 93 als effizient.

**Ausführungsbeispiel 4:** In eine Behälteraufnahme-Vorrichtung 7 mit vier Behälteraufnahmekammern 71 werden vier Glasspritzen entprechend dem obigen Ausführungsbeispiel 1, Format 1,25ml, eingebracht und über eine Haltevorrichtung fixiert. Die Glasspritzen werden in einer Vorrichtung 1 gemäß der in Fig. 10 dargestellten Vorrichtung 1 mit Durchlaufofen 45 zur Trocknung und Anbindung der Zwischenschicht mit einer zweischichtigen Beschichtung versehen. Analog zum Ausführungsbeispiel 1 werden sehr geringe Reibwerte erzielt.

### Ausführungsbeispiel 5:

Glasspritzen werden in eine Behälteraufnahme-Vorrichtung 7 gemäß der Abbildung 7 jeweils in die Öffnungen der beiden Außenelektroden eingeführt und analog zu Ausführungsbeispiel 1 mit einem Silikon-freien Fluid aus Fomblin M100 in Behandlungsstation 3 besprüht. Nachfolgend wird die Behälteraufnahme-Vorrichtung 7 zur Behandlungsstation 4 entsprechend Abbildung 7 befördert und über die Schließelemente 17, 18 werden die Spritzenkörper 25 gasdicht gegen äußeren Atmosphärendruck abgeschlossen und mit der Vakuumpumpe 190 und dem Gasverteiler 29 verbunden. Nach Vor-Evakuieren der Spritzenkörper 25 wird Prozessgas analog zum Ausführungsbeispiel 1 in die Spritzenkörper 25 eingeleitet. Durch Anlegen einer RadioFrequenz-Wechselspannung (RF-Spannung) mit einer Frequenz von 13,56 MHz an die beiden Außenelektroden wird in den Spritzenkörpern 25 simultan ein Plasma gezündet und damit die Silikon-freie Gleitschicht ausgehärtet.

### Bezugszeichenliste:

- 1: Vorrichtung zur Plasma-Behandlung
- 3, 4, 3A, 4A, 5,: Behandlungsstationen
- 7: Behälteraufnahme-Vorrichtung
- 9: Behandlungswerkzeug
- 11: Transporteinrichtung
- 12, 13, 14: Hubvorrichtung
- 15: Lanze
- 17, 18: Schließelement
- 19: Einrichtung zum Evakuieren der Spritzenzylinder
- 20: Vakuumverbindung
- 24: Dosierventil
- 25: Spritzenkörper
- 27: Wechselspannungs-Generator
- 29: Gasverteiler
- 31: Isolationselement
- 33: Lichtleiter
- 35: Photodetektor
- 37: Behälteraufnahme-Hülse
- 38: Aufnahme für 37
- 40, 41: Elektroden
- 42: Isolator zwischen 40, 41
- 43: Öffnung in 40, 41
- 45: Durchlaufofen
- 50: Zentriervorrichtung
- 51: Halterung von 50
- 52: Justierschraube
- 71: Behälteraufnahme-Kammer
- 72: offenes Ende von 71
- 91: Sprühdüse
- 92: Druckluftlanze
- 93: Heizdorn
- 111: Runddrehtisch
- 171, 172, 181: Dichtelement
- 175: Gasleitkanal
- 182: Gaseinlasskanal
- 190: Vakuumpumpe
- 191: Ventil
- 192: Absaugöffnung in 7
- 250: Spritzenzylinder
- 251: Innenseite des Spritzenzylinders
- 252: Kolbenöffnung
- 253: Flansch
- 254: Düse von 25
- 270: elektrische Kopplungseinrichtung

## Patentansprüche

1. Vorrichtung (1) zur Plasma-Behandlung von Behältern (250), mit folgenden Merkmalen:
- zumindest einer Behälteraufnahme-Vorrichtung (7) mit einer Behälteraufnahme-Kammer (71), die wenigstens ein offenes Ende (72) aufweist, durch welches ein zu behandelnder Behälter (250) einführbar ist, so dass eine Öffnung des Behälters (250) zum offenen Ende (72) der Behälteraufnahme-Kammer (71) weist;
- ein verschiebbares Schließelement (17), welches mit der Behälteraufnahme-Vorrichtung (7) über eine Hubvorrichtung (14) zusammenführbar ist, so dass beim Verschließen mittels der Hubvorrichtung (14) durch Bewegen des Schließelements (17) auf die Behälteraufnahme-Vorrichtung (7) zu ein aufgenommener Behälter (250) an einer Öffnung des Behälters (250) gegenüber dem äußeren Atmosphärendruck abgedichtet wird;
- eine Einrichtung (19) zum Erzeugen wenigstens einer gegenüber dem äußeren Atmosphärendruck abgeschlossenen Plasma-Zone mit zwei Elektroden (15, 7), über die ein elektrisches oder elektromagnetisches Feld im Inneren eines aufgenommenen und abgedichteten Behälters (250) erzeugbar ist, wobei die Einrichtung zum Erzeugen einer Plasma-Zone weiterhin - eine Einrichtung zum Entfernen des atmosphärischen Restgases aus dem Behälter (250) über eine gasdichte Verbindung am Schließelement (17) umfasst;
- zumindest ein Behandlungswerkzeug (9), das für wenigstens einen Teilschritt der Oberflächen-Behandlung der Behälter (250) mit dem offenen Ende der Behälteraufnahme-Kammer (71) der Behälteraufnahme-Vorrichtung (7) zusammenführbar ist, wobei das Behandlungswerkzeug (9) eine Vorrichtung zum Aufbringen eines organischen Films auf der Innenseite (251) eines Behälters (250) umfasst.

2. Vorrichtung gemäß Anspruch 1, wobei das Behandlungswerkzeug eine Sprühdüse (91) zum Aufsprühen eines organischen Flüssigkeitsfilms umfasst.

3. Vorrichtung gemäß einem der vorstehenden Ansprüche, zur Erzeugung einer Niederdruck-Plasma-Zone mit zwei Elektroden (15, 7), über die ein elektrisches oder elektromagnetisches Feld im Inneren eines aufgenommenen und evakuierten Behälters (250) erzeugbar ist, wobei die Einrichtung zum Erzeugen eines Niederdruck-Plasmas weiterhin
- eine Einrichtung (19) zum Evakuieren des Behälters (250) über
- eine Vakuumverbindung (20) am Schließelement (17) umfasst.

4. Vorrichtung gemäß einem der vorstehenden Ansprüche, wobei die Einrichtung zum Erzeugen einer Plasma-Zone eine Lanze (15) mit zumindest einem der folgenden Merkmale umfasst:
- die Lanze (15) bildet eine der beiden Elektroden, wobei diese Elektrode als Innenelektrode ausgebildet ist;
- die Lanze (15) bildet einen Gasverteiler zur Einführung und Verteilung von Prozessgas für das Niederdruck-Plasma;
- die Lanze (15) bildet eine Gasabsaug-Vorrichtung zum Absaugen von Prozessgas.

5. Vorrichtung gemäß einem der vorstehenden Ansprüche, **gekennzeichnet durch** mindestens eines der folgenden Merkmale:
- das Behandlungswerkzeug ist als Lanze ausgebildet, welche in das Behälterinnere eines in der Behälteraufnahme-Kammer aufgenommenen Behälters einführbar ist;
- die Vorrichtung zum Aufbringen eines organischen Films umfasst einen Vorratsbehälter mit einer in einem Plasma vernetzbaren organischen Substanz;
- zumindest zwei Behandlungsstationen und eine Transporteinrichtung zum Transport der Behälteraufnahme-Vorrichtung zwischen den Behandlungsstationen, wobei eine erste der Behandlungsstationen eine Behandlungsstation mit der Vorrichtung zum Aufbringen eines organischen Films auf der Innenseite eines Behälters ist und eine zweite der Behandlungsstationen in Transportrichtung der Transporteinrichtung nachgeordnet ist und die Einrichtung zum Erzeugen einer gegenüber dem äußeren Atmosphärendruck abgeschlossenen Plasma-Zone umfasst;
- eine Hubvorrichtung zum Verfahren des Behandlungswerkzeugs, um das Behandlungswerkzeug mit der Behälteraufnahme-Vorrichtung zusammenzuführen.

6. Vorrichtung gemäß einem der vorstehenden Ansprüche, eingerichtet zur Plasma-Behandlung von Behältern mit einem proximalen und einem distalen offenen Ende, vorzugsweise Spritzen oder Karpulen.

7. Vorrichtung gemäß vorstehendem Anspruch, zur Behandlung von vorzugsweise Zylinder-symmetrischen Behältern mit einer Längsachse, einem distalen Ende El und einem proximalen Ende E2 mit unterschiedlichen Innendurchmessern, wobei der Innendurchmesser DE1 des distalen Endes kleiner ist, als der Innendurchmesser DE2 des proximalen Endes, **dadurch gekennzeichnet, dass** durch die Vorrichtung die Behälter während des Sprühens mit dem Ende El mit dem kleineren Innendurchmesser nach oben zeigend entlang ihrer Längsachse im Bereich von 0° bis 45° zur Senkrechten, vorzugsweise im Bereich von -5° bis 5° orientiert werden.

8. Vorrichtung gemäß einem der vorstehenden Ansprüche, **gekennzeichnet durch** mindestens eines der folgenden Merkmale:
- die Behälteraufnahme-Vorrichtung umfasst wenigstens zwei vorzugsweise wenigstens vier Behandlungskammern zur simultanen Behandlung der Behälter;
- die Vorrichtung (1) umfasst eine weitere Behandlungsstation zur Vorbehandlung in Form einer Sprühstation, mit der eine Zwischenschicht innenseitig auf dem Behälter aufgebracht wird.

9. Verfahren zur innenseitigen Plasmabehandlung von Behältern (250) mittels einer Vorrichtung (1) gemäß einem der vorstehenden Ansprüche, bei welchem folgende Prozessschritte erfolgen:
- einen Behälter (25) mit Öffnung in das offene Ende (72) einer Behälteraufnahme-Kammer (71) einer Behälteraufnahme-Vorrichtung (7) einzusetzen, so dass die Öffnung des Behälters (250) zum offenen Ende der Behälteraufnahme-Kammer (71) weist,
- ein Behandlungswerkzeug (9) mit dem offenen Ende der Behälteraufnahme-Kammer (71) zusammenzuführen,
- Aufbringen eines organischen Films mittels des Behandlungswerkzeugs (9) auf der Innenseite des in die Behälteraufnahme-Kammer eingesetzten Behälters (250),
- Schließen des Behälters (250) an dessen Öffnung mittels Verschieben eines Schließelements (17), durch die Betätigung einer Hubvorrichtung (14), zum Zusammenführen des Schließelements (17) mit der Behälteraufnahme-Vorrichtung (7), und Abdichten des Behälters (250) mit dem Schließelement (17) gegenüber dem äußeren Atmosphärendruck,
- Entfernen des atmosphärischen Restgases des Behälterinneren mittels einer dafür geeigneten Einrichtung (190) über eine gasdichte Verbindung (20) am Schließelement,
- Erzeugen einer Plasmazone im Inneren des Behälters (250) und Behandeln des organischen Films mit dem Plasma.

10. Verfahren zur innenseitigen Plasmabehandlung von Behältern gemäß Anspruch 9, wobei das
- Evakuieren des Behälterinneren mittels einer Einrichtung zum Evakuieren des Behälters über eine Vakuumverbindung am Schließelement ausgeführt wird, und
- die erzeugte Plasmazone im Inneren des Behälters ein Niederdruck-Plasma ist zum Behandeln des organischen Films mit dem Niederdruck-Plasma.

11. Verfahren gemäß dem vorstehenden Anspruch, **dadurch gekennzeichnet, dass** der organische Film im Plasma vernetzt wird.

12. Verfahren gemäß einem der vorstehenden drei Ansprüche, **dadurch gekennzeichnet, dass** mindestens einer der folgenden Prozessschritte ausgeführt wird:
- durch eine Zentriervorrichtung wird die Behälteraufnahme-Vorrichtung zu den Behandlungswerkzeugen an den Behandlungsstationen in der Ebene senkrecht zur Hubrichtung der Behandlungswerkzeuge zentriert;
- ein Silikon-freier organischer Film wird aufgetragen, welcher Fluoralkyl-und/oder Ethylen-Gruppen aufweist, vorzugsweise in Form eines Fluids mit fluorierten oder perfluorierten Polyethern, und im gegenüber dem äußeren Atmosphärendruck abgeschlossenen Plasma vernetzt wird;
- durch ein Behandlungswerkzeug wird eine organische Gleitschicht auf der Innenseite des Behälters aufgebracht;
- durch das Verfahren erfolgt eine innenseitige Oberflächen-Behandlung oder Beschichtung von Behältern mit einem proximalen und einem distalen offenen Ende, vorzugsweise Spritzen oder Karpulen;
- wenigstens zwei, vorzugsweise vier Behälter werden simultan mittels separierter Plasma-Behandlungszonen behandelt.

13. Verfahren gemäß einem der vorstehenden Verfahrensansprüche **dadurch gekennzeichnet, dass** in einem vorherigen Schritt eine Zwischenschicht mittels eines Sprühprozesses auf der Behälter-Innenoberfläche aufgebracht wird.

14. Verfahren gemäß vorstehendem Anspruch **dadurch gekennzeichnet, dass** nach dem Aufbringen der Zwischenschicht ein Trocknungs- und/oder Aushärte-Schritt erfolgt, bei dem flüchtige Komponenten der Zwischenschicht aus dem Behälter-Innenraum entfernt werden, vorzugsweise durch einen nachfolgenden Behandlungsschritt in Form einer thermischen Behandlung, eines Sprühprozesses mit Druckluft oder in Form eines Evakuier-Prozesses.

15. Verfahren gemäß einem der vorstehenden Verfahrensansprüche, **dadurch gekennzeichnet, dass** mindestens einer der folgenden Prozessschritte ausgeführt wird:
- es werden vorzugsweise zylindersymmetrische Behälter mit einer Längsachse, einem distalen Ende E1 und einem proximalen Ende E2 mit unterschiedlichen Innendurchmessern behandelt, wobei der Innendurchmesser DE1 des distalen Endes kleiner ist, als der Innendurchmesser DE2 des proximalen Endes, **dadurch gekennzeichnet, dass** durch die Vorrichtung die Behälter während des Sprühens mit dem Ende El mit dem kleineren Innendurchmesser nach oben zeigend entlang ihrer Längsachse im Bereich von 0° bis 45° zur Senkrechten, vorzugsweise im Bereich von -5° bis 5° orientiert werden;
- die Orientierung der Behälter wird bei der nachfolgenden Plasma-Behandlung beibehalten.

## Claims

1. A device (1) for plasma treatment of containers (250), with the following features:
- at least one container receiving device (7) with a container receiving cavity (71) which has at least one open end (72) through which a container (250) to be treated is insertable in a manner so that an opening of the container (250) faces the open end (72) of the container receiving cavity (71);
- a displaceable closing element (17) which can be brought together with the container receiving device (7) by a lifting device (14), so that when closing by means of said lifting device (14) by moving the closing element (17) towards the container receiving device (7), a container (250) accommodated therein is sealed from the outer atmospheric pressure at an opening of the container (250);
- a means (19) for generating at least one plasma zone sealed from the outer atmospheric pressure, comprising a pair of electrodes (15, 7) across which an electric or electromagnetic field is generatable in the interior of an accommodated and sealed container (250), wherein said means for generating a plasma zone further comprises
- a means for removing the atmospheric residual gas from the container (250) via a gas-tight connection at the closing element (17);
- at least one treatment tool (9) which can be brought together with the open end of the container receiving cavity (71) of the container receiving device (7) for at least one sub-step of the surface treatment of the containers (250), said treatment tool (9) comprising a device for applying an organic film to the inner surface (251) of a container (250).

2. The device according to claim 1, wherein said treatment tool comprises a spray nozzle (91) for spray-depositing a liquid organic film.

3. The device according to any of the preceding claims, for generating a low-pressure plasma zone using a pair of electrodes (15, 7) across which an electrical or electromagnetic field is generatable inside an accommodated and evacuated container (250), wherein the means for generating a low-pressure plasma further comprises:
- a means (19) for evacuating the container (250) via
- a vacuum connection (20) at the closing element (17).

4. The device according to any of the preceding claims,
wherein the means for generating a plasma zone comprises a lance (15) with at least one of the following features:
- the lance (15) forms one of said pair of electrodes, said electrode being formed as an internal electrode;
- the lance (15) forms a gas manifold for introducing and distributing process gas for the low-pressure plasma;
- the lance (15) forms a gas suction device for extracting process gas.

5. The device according to any of the preceding claims, **characterized by** at least one of the following features:
- the treatment tool is formed as a lance which is introducible into the container interior of a container accommodated in the container receiving cavity;
- the device for applying an organic film comprises a reservoir with an organic substance cross-linkable in a plasma;
- at least two treatment stations and a transfer means for transferring the container receiving device between the treatment stations, wherein a first one of said treatment stations is a treatment station including the device for applying an organic film to the inner surface of a container, and a second one of the treatment stations is arranged downstream in the transfer direction of the transfer means and comprises the means for generating a plasma zone sealed from the outer atmospheric pressure;
- a lifting device for moving the treatment tool in order to bring together the treatment tool with the container receiving device.

6. The device according to any of the preceding claims, adapted for plasma treatment of containers having proximal and distal open ends, preferably syringes or carpules.

7. The device according to the preceding claim, for treating preferably cylindrically symmetric containers having a longitudinal axis, a distal end E1 and a proximal end E2 with different inner diameters, wherein the inner diameter DE1 of the distal end is smaller than the inner diameter DE2 of the proximal end, **characterized in that** the device is adapted to arrange the containers to have an orientation during the spraying with the end E1 having the smaller inner diameter facing upwards, with the longitudinal axes thereof in a range from 0° to 45° to the vertical, preferably in a range from -5° to 5°

8. The device according to any of the preceding claims, **characterized by** at least one of the following features:
- the container receiving device comprises at least two, preferably at least four treatment cavities for simultaneous treatment of the containers;
- the device (1) comprises a further treatment station for pre-treatment, which is configured as a spray-coating station which serves to apply an intermediate layer to the inner surface of the container.

9. A method for inside plasma treatment of containers (250) using a device (1) according to any of the preceding claims, comprising the method steps of:
- inserting a container (25) having an opening into the open end (72) of a container receiving cavity (71) of a container receiving device (7) in a manner so that the opening of the container (250) faces the open end of the container receiving cavity (71);
- bringing together a treatment tool (9) with the open end of the container receiving cavity (71);
- applying an organic film to the inner surface of the container inserted in the container receiving cavity (250) using the treatment tool (9);
- closing the container (250) at the opening thereof by displacing a closing element (17) by actuating a lifting device (14) for bringing together the closing element (17) with the container receiving device (7), and sealing the container (250) from the outer atmospheric pressure by means of said closing element (17);
- removing the atmospheric residual gas from the interior of the container using a suitable means (190) via a gas-tight connection (20) at the closing element;
- generating a plasma zone inside said container (250), and treating the organic film by said plasma.

10. The method for inside plasma treatment of containers according to claim 9, wherein the
- evacuating of the interior of the container is performed using a means for evacuating the container via a vacuum connection at said closing element; and
- the plasma zone generated inside the container is a low-pressure plasma for treating the organic film with the low-pressure plasma.

11. The method according to the preceding claim, **characterized in that** the organic film is cross-linked in the plasma.

12. The method according to any of the three preceding claims, **characterized in that** at least one of the following method steps is performed:
- centering the container receiving device with respect to the treatment tools at the treatment stations in the plane perpendicular to the lifting direction of the treatment tools using a centering device;
- applying a silicone-free organic film which comprises fluoroalkyl and/or ethylene groups, preferably in form of a fluid including fluorinated or perfluorinated polyethers, and cross-linking it in the plasma that is sealed from the outer atmospheric pressure;
- applying an organic lubricating layer to the inner surface of the container using a treatment tool;
- the method accomplishes an inside surface treatment or coating of containers that have proximal and distal open ends, preferably syringes or carpules;
- at least two, preferably four containers are treated simultaneously using separated plasma treatment zones.

13. The method according to any of the preceding method claims, **characterized in that** in a previous step an intermediate layer is applied to the inner surface of the container by a spraying process.

14. The method according to the preceding claim, **characterized in that** a drying and/or curing step is performed following said application of the intermediate layer, which removes volatile components of the intermediate layer from the interior of the container, preferably by a subsequent treatment step in form of a thermal treatment, a spraying process using compressed air, or in form of an evacuating process.

15. The method according to any of the preceding method claims, **characterized in that** at least one of the following method steps is performed:
- preferably cylindrically symmetric containers are treated which have a longitudinal axis, a distal end E1 and a proximal end E2 with different inner diameters, wherein the inner diameter DE1 of the distal end is smaller than the inner diameter DE2 of the proximal end, **characterized in that** during spraying the containers are oriented by said device with their ends E1 having the smaller inner diameter facing upwards and with their longitudinal axes in a range from 0° to 45° to the vertical, preferably in a range from -5° to 5°;
- the orientation of the containers is maintained during the subsequent plasma treatment.

## Revendications

1. Dispositif (1) pour le traitement plasma de récipients (250), comportant les caractéristiques suivantes :
- au moins un dispositif (7) pour la réception de récipients, comportant une chambre de réception de récipients (71), qui comporte au moins une extrémité (72) ouverte, à travers laquelle un récipient (250) à traiter peut être introduit de telle sorte qu'une ouverture du récipient (250) est dirigée vers l'extrémité (72) ouverte de la chambre de réception de récipients (71) ;
- un élément de fermeture (17) coulissant, qui peut être réuni au dispositif (7) pour la réception de récipients par l'intermédiaire d'un dispositif de levage (14), de telle sorte qu'au moment de la fermeture au moyen du dispositif de levage (14), sous l'effet du déplacement de l'élément de fermeture (17) vers le dispositif (7) pour la réception de récipients, un récipient (250) réceptionné est rendu étanche par rapport à la pression atmosphérique extérieure au niveau d'une ouverture du récipient (250) ;
- un dispositif (19) pour générer au moins une zone plasma fermée par rapport à la pression atmosphérique extérieure, comportant deux électrodes (15, 7) qui permettent de générer un champ électrique ou électromagnétique à l'intérieur d'un récipient (250) réceptionné et rendu étanche, ledit dispositif destiné à générer une zone plasma comportant, de plus, un dispositif pour éliminer le gaz résiduel atmosphérique hors du récipient (250) par l'intermédiaire d'une liaison étanche au gaz au niveau de l'élément de fermeture (17) ;
- au moins un outil de traitement (9) qui, pour au moins une étape partielle du traitement de surface des récipients (250), peut être réuni à l'extrémité ouverte de la chambre de réception de récipients (71) du dispositif (7) pour la réception de récipients, ledit outil de traitement (9) comportant un dispositif pour appliquer un film organique sur la face intérieure (251) d'un récipient (250) .

2. Dispositif selon la revendication 1, dans lequel l'outil de traitement comporte une buse de pulvérisation (91) pour pulvériser un film de liquide organique.

3. Dispositif selon l'une quelconque des revendications précédentes, destiné à générer une zone plasma basse pression avec deux électrodes (15, 7) qui permettent de générer un champ électrique ou électromagnétique à l'intérieur d'un récipient (250) réceptionné et mis sous vide, le dispositif destiné à générer une zone plasma basse pression comportant en outre :
- un dispositif (19) destiné à produire le vide dans le récipient (250) par l'intermédiaire
- d'une liaison sous vide (20) au niveau de l'élément de fermeture (17).

4. Dispositif selon l'une quelconque des revendications précédentes, dans lequel le dispositif destiné à générer une zone plasma comporte une lance (15) avec au moins une des caractéristiques suivantes :
- la lance (15) forme l'une des deux électrodes, ladite électrode étant réalisée sous forme d'électrode interne ;
- la lance (15) forme un répartiteur de gaz pour introduire et répartir le gaz du processus pour le plasma basse pression ;
- la lance (15) forme un dispositif d'aspiration du gaz pour aspirer le gaz du processus.

5. Dispositif selon l'une quelconque des revendications précédentes, **caractérisé par** au moins une des caractéristiques suivantes :
- l'outil de traitement est réalisé sous la forme d'une lance qui peut être introduite à l'intérieur d'un récipient logé dans la chambre de réception de récipients ;
- le dispositif destiné à appliquer un film organique comporte un réservoir de stockage contenant une substance organique réticulable dans un plasma ;
- au moins deux postes de traitement et un dispositif de transport pour transporter le dispositif de réception de récipients entre les postes de traitement, un premier des postes de traitement étant un poste de traitement comportant le dispositif destiné à appliquer un film organique sur la face intérieure d'un récipient, et un deuxième des postes de traitement étant disposé en aval du dispositif de transport dans la direction de transport et comportant le dispositif destiné à générer une zone plasma fermée par rapport à la pression atmosphérique extérieure ;
- un dispositif de levage pour déplacer l'outil de traitement en vue de réunir l'outil de traitement au dispositif de réception de récipients.

6. Dispositif selon l'une quelconque des revendications précédentes, configuré pour le traitement plasma de récipients munis d'une extrémité proximale ouverte et d'une extrémité distale ouverte, de préférence des seringues ou ampoules.

7. Dispositif selon l'une quelconque des revendications précédentes, pour le traitement de récipients de préférence cylindriques et symétriques ayant un axe longitudinal, une extrémité distale E1 et une extrémité proximale E2 avec des diamètres intérieurs différents, le diamètre intérieur DE1 de l'extrémité distale étant inférieur au diamètre intérieur DE2 de l'extrémité proximale, **caractérisé en ce que**, au moyen du dispositif, les récipients sont orientés pendant la pulvérisation avec l'extrémité E1 ayant le plus petit diamètre intérieur vers le haut le long de leur axe longitudinal dans une plage de 0° à 45° par rapport à la verticale, de préférence dans une plage de -5° à 5°.

8. Dispositif selon l'une quelconque des revendications précédentes, **caractérisé par** au moins une des caractéristiques suivantes :
- le dispositif de réception de récipients comporte au moins deux, de préférence au moins quatre, chambres de traitement pour le traitement simultané des récipients ;
- le dispositif (1) comporte un poste de traitement supplémentaire pour le traitement préliminaire sous la forme d'un poste de pulvérisation, au moyen duquel une couche intermédiaire est appliquée sur la face intérieure du récipient.

9. Procédé pour le traitement plasma à l'intérieur de récipients (250) au moyen d'un dispositif (1) selon l'une quelconque des revendications précédentes, dans lequel sont mises en oeuvre les étapes suivantes :
- introduire un récipient (25) avec ouverture dans l'extrémité (72) ouverte d'une chambre de réception de récipients (71) d'un dispositif (7) pour la réception de récipients, de telle sorte que l'ouverture du récipient (250) est dirigée vers l'extrémité ouverte de la chambre de réception de récipients (71),
- réunir un outil de traitement (9) avec l'extrémité ouverte de la chambre de réception de récipients (71),
- appliquer un film organique au moyen de l'outil de traitement (9) sur la face intérieure du récipient (250) inséré dans la chambre de réception de récipients,
- fermer le récipient (250) au niveau de son ouverture par le déplacement d'un élément de fermeture (17) sous l'effet de l'actionnement d'un dispositif de levage (14) destiné à réunir l'élément de fermeture (17) au dispositif (7) pour la réception de récipients, et étancher le récipient (250) par rapport à la pression atmosphérique extérieure au moyen de l'élément de fermeture (17),
- éliminer le gaz résiduel atmosphérique de l'intérieur du récipient au moyen d'un dispositif (190) prévu à cet effet par l'intermédiaire d'une liaison (20) étanche au gaz au niveau de l'élément de fermeture,
- générer une zone plasma à l'intérieur du récipient (250) et traiter le film organique avec le plasma.

10. Procédé pour le traitement plasma de l'intérieur de récipients selon la revendication 9, dans lequel :
- la production du vide à l'intérieur des récipients est effectuée au moyen d'un dispositif destiné à produire le vide dans le récipient par l'intermédiaire d'une liaison sous vide au niveau de l'élément de fermeture, et
- la zone plasma générée à l'intérieur du récipient est un plasma basse pression pour traiter le film organique avec le plasma basse pression.

11. Procédé selon la revendication précédente, **caractérisé en ce que** le film organique est réticulé dans le plasma.

12. Procédé selon l'une des trois revendications précédentes, **caractérisé en ce qu'**au moins une des étapes suivantes est mise en oeuvre :
- centrage du dispositif de réception de récipients au moyen d'un dispositif de centrage, par rapport aux outils de traitement au niveau des postes de traitement dans le plan perpendiculaire à la direction de levage des outils de traitement ;
- application d'un film organique exempt de silicone, lequel comporte des groupes fluoralkyle et/ou des groupes éthylène, de préférence sous la forme d'un fluide avec des polyéthers fluorés ou perfluorés, et réticulation dans le plasma fermé par rapport à la pression atmosphérique extérieure ;
- application d'une couche de glissement sur la face intérieure du récipient au moyen d'un outil de traitement ;
- mise en oeuvre, au moyen du procédé, d'un traitement de surface intérieur ou d'un revêtement intérieur des récipients munis d'une extrémité proximale ouverte et d'une extrémité distale ouverte, de préférence des seringues ou ampoules ;
- traitement simultané d'au moins deux, de préférence quatre, récipients au moyen de zones de traitement plasma séparées.

13. Procédé selon l'une quelconque des revendications de procédé précédentes, **caractérisé en ce que**, dans une étape précédente, une couche intermédiaire est appliquée sur la surface intérieure des récipients au moyen d'un processus de pulvérisation.

14. Procédé selon la revendication précédente, **caractérisé en ce que**, après l'application de la couche intermédiaire, est mise en oeuvre une étape de séchage et/ou de durcissement, dans laquelle des composants volatiles de la couche intermédiaire sont éliminés de l'intérieur du récipient, de préférence à l'appui d'une étape de traitement consécutive sous la forme d'un traitement thermique, d'un processus de pulvérisation à l'air comprimé ou sous la forme d'un processus de production du vide.

15. Procédé selon l'une quelconque des revendications de procédé précédentes, **caractérisé en ce qu'**au moins une des étapes suivantes du processus est mise en oeuvre :
- des récipients de préférence cylindriques et symétriques avec un axe longitudinal, une extrémité distale E1 et une extrémité proximale E2 ayant des diamètres intérieurs différents sont traités, le diamètre intérieur DE1 de l'extrémité distale étant inférieur au diamètre intérieur DE2 de l'extrémité proximale, **caractérisé en ce que**, au moyen du dispositif, les récipients sont orientés pendant la pulvérisation avec l'extrémité E1 ayant le plus petit diamètre intérieur vers le haut le long de leur axe longitudinal dans une plage de 0° à 45° par rapport à la verticale, de préférence dans une plage de -5° à 5° ;
- l'orientation des récipients est maintenue pendant le traitement plasma consécutif.
